# EUROPÄISCHE PATENTSCHRIFT

(11) **EP 3 076 981 B1**
(45) Veröffentlichungstag und Bekanntmachung des Hinweises auf die Patenterteilung: **24.10.2018**
(21) Anmeldenummer: 13799053.7
(22) Anmeldetag: 02.12.2013
(51) Int. Cl.: A61K 35/20, A61K 31/593, A61P 35/00

(54) **PHARMAZEUTISCHE ZUSAMMENSETZUNG ZUR PROPHYLAXE UND/ODER BEHANDLUNG VON ERKRANKUNGEN MIT EINER GESTÖRTEN LPS- UND/ODER APOPTOSE-REGULATION**
PHARMACEUTICAL COMPOSITIONS FOR THE PROPHYLAXIS AND/OR TREATMENT OF DISORDERS WITH A DISTURBED REGULATION OF LPS- AND/OR APOPTOSIS
COMPOSITION PHARMACEUTIQUE POUR LA PROPHYLAXIE ET/OU LE TRAITEMENT DE MALADIES ACCOMPAGNÉES D'UNE RÉGULATION PERTURBÉE DU LPS ET/OU DE L'APOPTOSE

(43) Veröffentlichungstag der Anmeldung: 12.10.2016
(73) Patentinhaber: Julius-Maximilians-Universität Würzburg, 97070 Würzburg (DE)
(72) Erfinder: GASSER, Martin, 97080 Würzburg (DE); LISSNER, Reinhard, 63937 Weilbach (DE); WAAGA-GASSER, Ana, Maria, 97080 Würzburg (DE); WOLZ, Georg, 65366 Geisenheim (DE)
(74) Vertreter: Patentanwälte Gierlich & Pischitzis Partnerschaft mbB
(86) Internationale Anmeldenummer: PCT/EP2013/075287
(87) Internationale Veröffentlichungsnummer: WO 2015/081982

(56) Entgegenhaltungen:
- WO-A1-98/00149
- WO-A1-2008/079030
- WO-A2-2008/140335
- DATABASE WPI Week 201211 Thomson Scientific, London, GB; AN 2011-Q64192 XP002728159, & CN 102 239 921 A (JIANGSU XINGCHI BIO TECHNOLOGY CO LTD) 16. November 2011 (2011-11-16)

## Beschreibung

### Gebiet der Erfindung

Die Erfindung betrifft eine pharmazeutische Zusammensetzung zur Behandlung von Erkrankungen mit einer gestörten LPS- und/oder Apoptose-Regulation.

Das Kolostrum (lat. colostrum, auch Colostrum) ist die Erstmilch für Säugetiere, die von zahlreichen Säugetierspezies peripartal produziert wird um das Neugeborene in den ersten Tagen optimal zu ernähren. Es wird auch als Vormilch, Kolostralmilch, Biest bzw. Biestmilch (beides bei Kühen) oder Erstmilch bezeichnet und besteht u.a. aus Proteinen, Enzymen, Vitaminen, Mineralien, Wachstumsfaktoren, Aminosäuren und Antikörpern. Auf diese Weise wird die Stärkung und die Immunabwehr des Jungtiers oder Kindes als eine besondere Form von Leihimmunität unterstützt bzw. ersetzt, bis ein eigenes Immunsystem nach einigen Tagen bis Wochen etabliert ist.

Das Kolostrum von Kühen gilt als gesundes Lebensmittel. Es gibt zahlreiche Studien zur Wirksamkeit von Kolostrum. Unter anderem soll Kolostrum einen gewissen Schutz vor Infektionskrankheiten bieten (Cesarone, et al.: Prevention of influenza episodes with colostrum compared with vaccination in healthy and high-risk cardiovascular subjects: The epidemiologic study in San Valentino: Clinical and Applied Thrombosis/Hemostasis 13 (2) / 2007, pp. 130-136; Tacket et al.: Efficacy of bovine milk immunoglobulin concentrate in preventing illness after Shigella flexneri challenge: American Journal Tropical Medicine Hygiene 47(3) / 1992, pp.276-83;Huppertz et al.: Bovine Colostrum ameliorates diarrhea in infection with diarrheogenic E.coli, Shiga Toxin-producing E.coli and E.coli Expressing Intimin and Hemolysin J Ped Gastroenterol Nutr 29: 452-456/1999) und die Wundheilung sowie die Regeneration geschädigter Darmschleimhaut unterstützen (Uruakpa et al.: Colostrum and its benefits: a review: Nutrition Research 22 / 2002, pp.755-67). Kolostrum wird auch zur Mitbehandlung von Allergien, Bluthochdruck, Diabetes mellitus und Depressionen eingesetzt. Belege für die Wirksamkeit der Anwendung bei diesen Indikationen fehlen jedoch bisher.

Cholecalciferol (auch Colecalciferol oder kurz Calciol), Vitamin D3, ist das physiologisch in allen nichtpflanzlichen Eukaryoten vorkommende Vitamin D. Es wird im Körper mit Hilfe von ultraviolettem Licht (UV-B) in der Haut aus 7-Dehydrocholesterol gebildet.

In der Nahrung kommt es vor allem in Fettfischen (als Fettfische werden - im Gegensatz zur Gruppe der in der Fischerei Weißfisch genannten fettärmeren Sorten wie Kabeljau oder Schellfisch - Speisefische bezeichnet, in deren Muskelgewebe das Fett einen Anteil von etwa zwei Prozent übersteigt; er kann - je nach Art - über 30 Prozent betragen. Bekannte Arten sind z.B. Hering, Sprotte, Sardine und Sardelle, Lachs, Makrele, Thunfisch, Aal und Karpfen. Meerlebende Fettfische gelten wegen ihres Gehalts an Omega-3-Fettsäuren als ernährungsphysiologisch wertvoll) vor oder wird den Lebensmitteln als Nahrungsergänzungsmittel zugefügt. Es hat im Körper die Funktion eines Prohormons und wird über eine Zwischenstufe zu dem Hormon Calcitriol umgewandelt.

Calcitriol, auch 1α,25(OH)₂-Cholecalciferol (1α,25(OH)₂Vitamin D3) oder kurz 1,25(OH)₂D3, ist ein hochwirksames Secosteroid mit struktureller Ähnlichkeit zu den Steroidhormonen. Es ist - wie dargelegt - die physiologisch aktive Form des Prohormons Vitamin D3. Es wird durch die 1α-Hydroxylase vor allem in den Nieren, aber auch in anderen Geweben aus dem 25(OH)Vitamin D3 hydroxyliert oder in seltenen Fällen als Medikament verordnet.

Calcitriol wird an ein intrazelluläres Rezeptorprotein, den Vitamin-D-Rezeptor (VDR) gebunden und in den Zellkern transportiert. Dort assoziiert der Vitamin-Rezeptor-Komplex an die DNA und verändert die Transkription verschiedener hormonsensitiver Gene, was schließlich zu Änderungen in der Proteinsynthese mit entsprechenden biologischen Wirkungen führt.

Vitamin D spielt u.a. eine wesentliche Rolle bei der Regulierung des Calcium-Spiegels im Blut und beim Knochenaufbau. Ein Vitamin D-Mangel führt mittelfristig zu diversen Erkrankungen, bspw. zu Rachitis oder Osteomalazie.

Lactoferrin (genauer: Lactotransferrin, aus lateinisch lacteus Milch und lat. ferrum Eisen und lat. transferre hinübertragen) ist ein in Säugetieren vorkommendes Protein mit multifunktionalen Enzymaktivitäten.

Lactoferrin gehört zur Proteinfamilie der Transferrine. Transferrine kommen nicht nur bei Säugetieren vor, homologe Gene finden sich auch bei anderen Wirbeltieren und Wirbellosen.

Lactoferrin ist in vielen Körperflüssigkeiten der Säugetiere, in deren Milch, Tränen, Speichel, Schweiß, Vaginalsekret, Seminalplasma, Nasen- und Bronchialsekret sowie anderen Sekretionen zu finden. Außerdem ist es in weißen Blutkörperchen lokalisiert.

Lactoferrin besitzt sowohl antivirale (Harmsen, Martin C. et al.: Antiviral effects of plasma and milk proteins: lactoferrin shows potent activity against both human immunodeficiency virus and human cytomegalovirus replication in vitro. Journal of Infectious Diseases, Band 172, Nr. 2, 1995, S. 380-388) als auch antimikrobielle (Park, Ji-Hye et al.: An antimicrobial protein, lactoferrin exists in the sweat: proteomic analysis of sweat. Experimental Dermatology, Band 20, Nr. 4, 2011, S. 369-371) Eigenschaften. Es fungiert einerseits als Peptidase (Spaltung von Peptiden), weshalb es der Gruppe der Serinproteasen zugeordnet wird, aber auch als Eisen-bindendes Protein - ähnlich dem Transferrin - und zeigt zusätzlich Desoxyribonuklease- und Ribonuklease-Aktivitäten, womit es den Nukleasen (EC 3.1.21.1) zugerechnet wird. Außerdem ist es ein starker Inhibitor für Tryptase.

Ein Liter Kuhmilch enthält ca. 0,1 g Lactoferrin. Es ist nur zu einem geringen Teil mit Eisen gesättigt und kann mehr als das Fünffache seiner ursprünglichen Eisenladung binden.

Industriell wird es aus Milch und Molke isoliert. Es wird in Baby- und Sportlernahrung, sowie in Kosmetika, Kaugummis und Functional Foods eingesetzt.

Vom Organismus wird Lactoferrin u.a. eingesetzt, um Bakterien das lebensnotwendige Eisen zu entziehen. Da Bakterien essentiell auf Eisen angewiesen sind, wirkt die Eisenverarmung antibakteriell. Als Protease ist Lactoferrin in der Lage, mehrere für die Besiedlung wichtige Proteine des Krankheitserregers *Haemophilus influenzae* zu zerstören. Außerdem beeinträchtigt es in *Shigella* und pathogenen *Escherichia coli* das Typ III-Sekretionssystem. Von Leukozyten freigesetzt, ist es so auch Teil des Immunsystems.

### Stand der Technik

Kolostrum bzw. Kolostrumpräparate werden in mannigfaltiger Weise und Aufbereitung kommerziell angeboten. So sind bspw. Kolostrumpräparate als Flüssigkeit, in Kapselform, als Colobons oder als Hautbalsam käuflich erhältlich.

Ein Anbieter von Kolostrumpräparaten ist die Firma Dr. Wolz Zell GmbH, Marienthaler Str. 3, 65366 Geisenheim (www.wolz.de), die unter dem Markennamen Lactobin® N ein Kolostrumpräparat in Pulverform anbieten.

Von diesem Lactobin® N bzw. seinem bioäquivalenten Vorläuferpräparat Lactobin® Biotest ist seit längerem bekannt, dass die in den Präparaten vorkommenden Antikörperspezifikationen gegen bakterielle Lipopolysaccharide wirken.

Diese Befunde des Einsatzes von Lactobin® N führten zu präklinischen und klinischen Studien, die in zwei Übersichtspublikationen von W. Struff und G. Sprotte beschrieben wurden (W.G. Struff and G. Sprotte: Bovine colostrum as a biologic in clinical medicine: a review. International Journal of Clinical Pharmacology and Therapeutics, Vol. 45 - No. 4/2007 (193-202); W.G. Struff and G. Sprotte: Bovine colostrum as a biologic in clinical medicine: A review - Part II: International Journal of Clinical Pharmacology and Therapeutics, Vol. 46 - No. 5/2008 (211-225) .

Auf der Basis eines Zufallsbefundes von G. Sprotte bezüglich gestörter Apoptosefunktionen mononukleärer Zellen im Blut von Patienten mit chronisch rezidivierenden Schmerzzuständen wurden Patienten während und auch außerhalb ihrer typischen Schmerzschübe erfolgreich behandelt, wodurch die kausale Beziehung zwischen Schmerzentstehung und -Kupierung durch Lactobin® N als zusammenhängend mit der durch die Gabe des Präparates normalisierten Apoptosefunktion erkannt wurde (A.M. Waaga-Gasser et al.: Oral immunoglobulin induces mononuclear cell apoptosis in patients suffering from idiopathic chronic pain syndrome: Results from a pilot study. International Journal of Clinical Pharmacology and Therapeutics, Vol. 47 - No. 7/2009 (421-433).

Proinflammatorische Zytokine in dem untersuchten peripheren Blut waren während eines Schmerzschubes auffällig erhöht, während antiinflammatorische Zytokinprofile als erniedrigt gemessen wurden. Unter der Behandlung mit Lactobin® N kam es mit dem gleichzeitigen Wiederauftreten der Schmerzen im Falle eines bewusst herbeigeführten Auslassversuches zur Umkehrung dieser Profile. Auch der zusätzlich bestimmte Serumspiegel des Insulin like Growth Factor I (IGF I) zeigte sich in der behandlungsfreien Phase signifikant erhöht und fiel unter Lactobin® N auf Normalniveau ab. Auch dieses Phänomen wird von den Untersuchern eindeutig in einem Zusammenhang mit der Apoptosedysfunktion und der Schmerzentstehung bzw. der Schmerzstillung unter Lactobin® N gesehen. Ein analoger Befund von extraintestinaler Fernwirkung des Kolostrumpräparates auf andere Körperkompartimente war erstmalig in einer klinischen Studie mit dem Vorgängerpräparat (Lactobin® Biotest) beobachtet worden, insofern es unter der Behandlung bauchchirurgischer Patienten zu einer signifikanten Senkung des peripher erhöhten LPS-Spiegels (im Vergleich zur Placebogruppe) im Serum gekommen war (Edwin Bölke et al.: PREOPERATIVE ORAL APPLICATION OF IMMUNOGLOBULIN-ENRICHED COLOSTRUM MILK AND MEDIATOR RESPONSE DURING ABDOMINAL SURGERY. SHOCK, Vol. 17, No. 1, pp. 9-12, 2002).

Weiterhin besteht bei vielen Patienten mit verschiedenartigen Tumorentitäten eine Störung der Apoptose in immunkompetenten Zellen, was ein wesentliches zugrundeliegendes Problem bei fortschreitender Tumorerkrankung darstellt. Der verstärkte Untergang von Immunzellen wird durch eine Dysregulation von LPSgetriggerten, chronisch erhöhten Entzündungsabläufen (chronische Inflammation) aufrechterhalten. Relevante pathophysiologische Erkenntnisse liegen dazu erst seit kurzem vor und wurden vor allem durch die Erforschung der Toll Like Rezeptoren (TLR) substantiiert. In diesem Zusammenhang ist auf die präklinischen Versuchsergebnisse von Fitzal et al. (Florian Fitzal et al.: Immunoglobulin Enriched Colostral Milk Reduces Gut-Derived Endotoxemia in a Rat Hemorrhage Model. European Journal of Trauma, 2001, pp. 257-263) und die schon erwähnten Ergebnisse der klinischen Phase II Studie mit Lactobin® N (Edwin Bölke et al.: PREOPERATIVE ORAL APPLICATION OF IMMUNOGLOBULIN-ENRICHED COLOSTRUM MILK AND MEDIATOR RESPONSE DURING ABDOMINAL SURGERY. SHOCK, Vol. 17, No. 1, pp. 9-12, 2002) hinzuweisen. Diese Befunde legen den Schluss nahe, dass das Kolostrumpräparat durch intraenterale LPS-Neutralisation den gastrointestinalen Übertritt des Bakterientoxins von der Darmoberfläche in zentrale Körperkompartimente reduziert. Denaturierungsprozesse und ein teilweiser proteolytischer Abbau der bovinen Proteinmoleküle finden zwar im Magendarmtrakt statt, aber zumindest 20% der eingenommenen Menge des bovinen IgGs erreichen die Ileocoecalklappe in vermutlich aktiver Form (N. ROOS et al.: 15N-Labeled Immunoglobulins from Bovine Colostrum Are Partially Resistant to Digestion in Human Intestine. The Journal of Nutrition, 1995, pp. 1238-1244; R. Lissner et al.: Antibody reactivity and fecal recovery of bovine immunoglobulins following oral administration of a colostrum concentrate from cows (Lactobin) to healthy volunteers. International Journal of Clinical Pharmacology and Therapeutics., Vol. 36, No. 5 - 1998 (239-245)).

Weiterhin haben verschiedene epidemiologische Studien Hinweise auf eine Korrelation zwischen der Höhe der Calcitriol-Plasmaspiegel von Patienten und deren Inzidenz für verschiedene Karzinome erbracht (Stubbins RE et al 2012. Using components of the vitamin D pathway to prevent and treat cancer. Nutrition Reviews 70:721-729; Manson J E. 2011. Vitamin D and Prevention of Cancer - Ready for Prime Time?. NEJM 364(15): 1385-87; Pietschmann P. et al. 2003. Bedeutung von Vitamin D im Immunsystem. J Mineral Stoffwechs 10(3): 13-15; BMJ Research. Association between pre-diagnostic circulating Vitamin D concentration and risk of colorectal cancer in European Populations: A nested case-control studyBMI/OnlineFirst/bmj.com).

Zurückliegende Untersuchungen geben Hinweise für eine Einflussnahme Vitamin D-Rezeptor-vermittelter intrazellulärer Mechanismen auf die entscheidenden deregulierten wnt/ß-catenin Signalwege in Tumorzellen. Weiterhin scheint Vitamin D3 durch Kooperation mit anderen regulatorischen Signalwegen über den humanen Vitamin D-Rezeptor als einen entscheidenden nukleären Rezeptor in der (Tumor-)Zelle immunregulatorische und antitumorigene Effekte sowie antimikrobielle und detoxifizierende Effekte zu entfalten (Haussler MR et al. Vitamin D receptor: molecular signaling and actions of nutritional ligands in disease prevention. Nutritional Reviews 2008; 66:98-112).

Ferner wurde jüngst deutlich, dass in Tumorzellen eine deutlich erniedrigte Vitamin D-Rezeptor-Dichte vorliegt. Die Gründe hierfür bleiben bislang weitestgehend unbekannt; sie machen aber therapeutische Strategien zur Erhöhung der Vitamin D-Spiegel und seiner Rezeptorendichte auf Tumorzellen umso bedeutsamer.

Aus der Patentliteratur sind einige Schriften bekannt, die den Einsatz von Kolostrum bzw. eines Kolostrumpräparates - oder einzelner biologisch aktiver Bestandteile daraus - beschreiben.

So wird bspw. in der EP 0917467 A1 die Behandlung von entzündlichen Darmerkrankungen mit Kolostrum beschrieben. Ein Hinweis auf die Zugabe weiterer Wirkstoffe als Kombinationstherapie oder der Behandlung spezieller Erkrankungen ist dem Dokument nicht zu entnehmen.

Die EP 2121002 A1 beschreibt u.a. die Verwendung von Lactoferrin oder Lactoferrin-Metallionen - konkret durch nachträgliche Behandlung zu 100% eisengesättigtes Lactoferrin - sowie mindestens einen Anti-Tumor-Lebensmittelfaktor wie beispielsweise Soyaprotein zur Behandlung von Krebs. Die Verwendung des - alle biologisch aktiven Bestandteile aufweisenden - Kolostrums bzw. eines Kolostrumpräparates ohne notwendige Eisensättigung sowie die Zugabe weiterer Wirkstoffe als Kombinationstherapie wird nicht beschrieben.

### Aufgabe

Aufgabe der Erfindung ist es, eine pharmazeutische Zusammensetzung bereitzustellen, die eine Behandlung von Erkrankungen mit einer gestörten LPS- und/oder Apoptose-Regulation ermöglichen, nämlich die Behandlung von Tumoren des Gastrointestinaltraktes.

Weiterhin ist es Aufgabe der Erfindung, die pharmazeutische Zusammensetzung für die Verwendung zur Behandlung von diesen Erkrankungen mit einer gestörten LPS- und/oder Apoptose-Regulation bereitzustellen.

### Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht. Die Erfindungen umfassen auch alle sinnvollen und insbesondere alle erwähnten Kombinationen von unabhängigen und/oder abhängigen Ansprüchen.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen. Überraschenderweise wurde gefunden, dass eine pharmazeutische Zusammensetzung enthaltend Kolostrum oder ein Kolostrumpräparat und Vitamin D zur Prophylaxe und/oder zur Behandlung von Erkrankungen mit einer gestörten LPS- und/oder Apoptose-Regulation genutzt werden kann.

Neueren Literaturangaben zufolge kommen im Rohkolostrum, bspw. Rohkolostrum bovinen Ursprungs, nur die Vitamine A, B12 und E in geringen Konzentrationen vor (A.M. Keech: Peptide Immuntherapy - Colostrum - A Physician's Reference Guide. AKS Publishing 2010). Ferner ist davon auszugehen, dass die fettlöslichen Vitamine (A, D, E, K), zumindest im Herstellungsprozess von allen Kolostrumkonzentraten, im Zuge der Lipoproteinabtrennung mitentfernt werden. Sie können im Endprodukt in Spuren vorhanden sein (A M Keech Peptide Immunotherapy. Colostrum -A Physician's Reference Guide AKS Publishing 2010, pp 84). Bei der erfindungsgemäßen pharmazeutischen Zusammensetzung reicht daher das ggf. natürlicherweise im Kolostrum enthaltene Vitamin D bzw. Vitamin D3 keinesfalls aus, um die gewünschte immunregulatorische Wirkung zu entfalten. Es muss daher der pharmazeutischen Zusammensetzung hinzugegeben werden.

Anders verhält es sich mit dem Lactoferrin bzw. Lactotransferrin. Dieses ist ein natürlicherweise vorkommender Inhaltsstoff des Kolostrums und liegt diesem erfindungsgemäß in Mengen vor, die ausreichend sind die erwünschte pharmazeutische Wirkung - zusammen mit den weiteren natürlicherweise im Kolostrum vorkommenden Inhaltsstoffen und dem (hinzugegebenen) Vitamin D3 - zu entfalten. Der erfindungsgemäßen pharmazeutischen Zusammensetzung wird daher Lactoferrin bzw. Lactotransferrin nicht hinzugegeben.

Durch die gezielte und neue Kombination einzelner, aus der Human- und der Veterinärmedizin bekannter, Wirkstoffe konnte erfindungsgemäß gezeigt werden, dass die Neutralisation von Lipopolysacchariden (LPS) im Magendarmtrakt von Säugetieren und die Rückführung der von LPS ausgelösten und chronisch unterhaltenen subklinischen Entzündungszustände, eine zentrale Rolle für die Pathophysiologie septischer Zustände, des chronisch rezidivierenden Schmerz-Syndroms und vor allem für die Onkogenese darstellt.

Völlig unerwartet konnte der Nachweis einer Wirkungssteigerung durch den Einsatz einer Kombination einzelner Wirkstoff (komponenten) gezeigt werden. Dieser nachgewiesene Synergismus zeigte eine Normalisierung der wichtigsten Komponenten der durch LPS ausgelösten Entzündungskaskade. Vor allem die Restitution der supprimierten Apoptosekapazität in periphervenösen Monozyten und der Entzündungszellen in dem untersuchten, intraoperativ entnommenen Tumorgewebe von Patienten mit CRC stellt einen antineoplastischen Schlüsseleffekt, insbesondere um den Operationszeitpunkt (d. h. perioperativ) bei der Behandlung von Tumorpatienten (einer der vier Haupttypen zur Therapie von Tumor- bzw. Krebspatienten, neben der Strahlentherapie, der Chemotherapie und der gerichteten Therapie mittels monoklonalen Antikörpern) sowie während der chemotherapeutischen Phasen, dar.

Angesichts der Tatsache, dass einer der eingesetzten Wirkstoffe, nämlich Vitamin D3, seine Wirkungen grundsätzlich durch die Aktivierung von spezifischen Rezeptoren entfaltet, konnte gezeigt werden, dass die Kombination der Wirkstoffe eine qualitativ unterschiedliche Immunantwort auszulösen vermag insofern als vor allem gestörte Apoptoseabläufe in Immunzellen von CRC-Patienten (insbesondere periphervenöse Monozyten und solche welche massenweise Tumorgewebe infiltrieren) ex vivo revitalisiert und durch Inkubation mit den hier verwendeten Wirkstoffen und deren Kombination normalisiert werden können.

In einer bevorzugten Ausführungsform der pharmazeutischen Zusammensetzung ist diese dadurch gekennzeichnet, dass das darin enthaltene Vitamin D als Vitamin D3 (Prohormon: Cholecalciferol) und/oder als seine biologisch aktive Form Calcitriol vorliegt, bevorzugt als Cholecalciferol.

In einer weiteren bevorzugten Ausführungsform der pharmazeutischen Zusammensetzung zeichnet sich diese dadurch aus, dass das darin enthaltene Vitamin D, vorzugsweise Vitamin D3 als Cholecalciferol und/oder als Calcitriol, 0,00001-0,5 Gew.% der pharmazeutischen Zusammensetzung ausmacht. Bevorzugt ist ein Gehalt von 0,00002 bis 0,4 Gew.-%, besonders bevorzugt zwischen 0,00002 bis 0,3 Gew.-%. Ganz besonders bevorzugt ist ein Gehalt zwischen 0,00001 Gew.% und 0,001 Gew.% oder 0,1 µg/g und 10 µg/g, insbesondere zwischen 0,00001 Gew.% und 0,0005 Gew.% oder 0,1 µg/g und 5 µg/g, insbesondere zwischen 0,00001 Gew.% und 0,001 Gew.% oder 0,1 µg/g und 1 µg/g. Als besonders bevorzugt gilt ein Gehalt zwischen 0,4 µg/g und 0,6 µg/g.

In einer weiteren bevorzugten Ausführungsform der Erfindung zeichnet sich die pharmazeutische Zusammensetzung dadurch aus, dass das Kolostrum von Säugetieren ausgewählt aus der Gruppe der Einhufer (*Equus;* bspw. Pferde) oder Paarhufer (*Artiodactyla* oder *Paraxonia;* bspw. Rinder, Schweine, Kamele, Ziegen, Schafe) stammt. Vorzugsweise ist die pharmazeutische Zusammensetzung dadurch gekennzeichnet, dass das darin enthaltene Kolostrum von Wiederkäuern (*Ruminantia;* bspw. Rinder, Ziegen, Schafe) stammt. Besonders bevorzugt ist es, wenn das Kolostrum von Rindern (*Bovini*) stammt. Hinzu kommt auch, dass Milchkühe in der Regel zu einer Überproduktion von Kolostrum neigen, welches von den neugeborenen Kälbern nicht gänzlich verbraucht wird.

Bevorzugt stammt das Kolostrum von Rindern, welche nicht in Stallhaltung gehalten werden. Solche Tiere sind weniger anfällig für Infektionskrankheiten. Weiterhin zeichnet sich das Kolostrum derartiger Rinder, insbesondere australischer oder neuseeländischer Rinder, dadurch aus, dass etwaige saisonal bedingte Unterschiede des Gehalts an Bioaktivatoren, wie dies bspw. in Produktionschargen europäischer Rinder, die häufig, bspw. während der Wintermonate, in Stallungen gehalten werden, beobachtet wurde, bisher nicht gemessen werden konnten.

In einer weiteren bevorzugten Ausführungsform der Erfindung stammt das Kolostrum aus mindestens 100, vorzugsweise mindestens 200, Einzelkolostren. Ein aus vielen Einzelkolostren gewonnenes Kolostrum zeigt gegenüber aus wenigen Einzelkolostren gewonnenes Kolostrum viele Vorteile. Es ist z.B. polyvalent, d.h. es zeigt ein hohes Maß an Homogenität und die Konzentration der Wirkstoffe bzw. deren Aktivitäten sind chargenunabhängig. Es kann gemäß der EU-GMP-Richtlinien (Richtlinien zur Qualitätssicherung bei Produktion, Equipment, Medien und Reinraumumgebung in der Produktion von Arzneimitteln und Wirkstoffen, Kosmetika, Lebens- und Futtermitteln) hergestellt werden. Hyperimmunkonzentrate hingegen sind aus wenigen Einzelkolostren, häufig von vakzinierten Muttertieren (z.B. Kühen) gewonnene Präparate, die zum Einsatz bei Patienten mit differentialdiagnostisch abgeklärten Infektionskrankheiten kommen. Vorzugsweise wurde das erfindungsgemäße Kolostrum innerhalb der ersten 24 Std. bis 72 Std. nach der Geburt produziert und abgegeben, besonders bevorzugt innerhalb der ersten 48 Std., insbesondere innerhalb der ersten 24 Std., nach der Geburt. Es ist von großer Bedeutung, dass das Kolostrum innerhalb dieser Zeiträume erhalten wird, da sich der Gehalt an Zytokinen im Kolostrum nach der Geburt stark ändern kann.

Die erfindungsgemäße pharmazeutische Zusammensetzung kann grundsätzlich in allen pharmazeutisch sinnvollen und dem Fachmann bekannten Verabreichungsformen vorliegen, bspw. als Pulver, Tabletten, Kapseln, Spray, Flüssigkeit oder Zäpfchen. Es kann in Reinform, aber auch als Konzentrat, z.B. als Pulverkonzentrat, einer besonders bevorzugten Ausführungsform der Erfindung, vorliegen. Es kann darüber hinaus noch alle zur Nutzung als pharmazeutische Zusammensetzung und gemäß der gewählten Applikations- bzw. der Verabreichungsform (bspw. endobronchial, enteral, epidural, inhalativ, intraarteriell, intraartikulär, intragastral, intrakardial, intrakutan, intralumbal, intralymphatisch, intramammär, intramuskulär, intraneural, intraperitoneal, intrapleural, intrapulmonal, intratracheal, intraurethral, intrathekal, intravenös, intranasal, oral (dort auch lokal wirksam, wenn es bspw. einige Minuten lang in der Mundhöhle gehalten wird und anschließend damit gegurgelt wird. Das bewährte sich bisher bei ersten Anzeichen von Tonsillitis, Pharyngitis, Laryngitis. Im Prodromalstadium von Virusgrippe kam es bei Patienten zu einer sofortigen Beseitigung der subjektiven Symptome wie Fieber , Glieder/Rückenschmerzen oder Abgeschlagenheit) peroral, parenteral, perkutan, peridural, perineural, rektal, sublingual, subkonjunktival, subkutan, systemisch, topisch, transdermal, vaginal) notwendigen und dem Fachmann bekannten Füllstoffe und Additive, bspw. Füllmittel, Bindemittel, Sprengmittel, Gleitmittel enthalten und z.B. auch in gefriergetrocknetem Zustand vorliegen.

In einer bevorzugten Ausführungsform werden die Kolostren entfettet, filtriert und pasteurisiert (z. B. bei 72 °C für 15 s) bevor durch Trocknen, bevorzugt Sprühtrocknen, ein Pulver hergestellt wird.

In einer weiteren bevorzugten Ausführungsform der Erfindung weist das Kolostrum der pharmazeutischen Zusammensetzung, vorzugsweise wenn dieses als Pulverkonzentrat vorliegt, 70-90 Gew.% Protein und weniger als 5 Gew.% Fett auf.

In einer weiteren bevorzugten Ausführungsform weist das Kolostrum der pharmazeutischen Zusammensetzung 70- 90 Gew.% Protein, weniger als 5 Gew.% Fett und weniger als 0,2 µg/100 g Vitamin D auf.

In einer weiteren bevorzugten Ausführungsform weist das Kolostrum der pharmazeutischen Zusammensetzung 75-90 Gew.% Protein, weniger als 2 Gew.% Fett und weniger als 0.2 µg/100 g Vitamin D auf.

Statt weniger als 2 Gew.% kann der Gehalt an Fett auch zwischen 0,5 und 2 Gew.% liegen.

In einer weiteren bevorzugten Ausführungsform enthält der Proteinanteil der vorstehend genannten Kolostren vorzugsweise über 20 Gew.% IgG (gemessen mit HPLC), bevorzugt 20-30 Gew.%, besonders bevorzugt zwischen 20 und 24 Gew.%, vorzugsweise IgG der Subklasse IgG1 und 1-5 Gew.% Lactoferrin bzw. Lactotransferrin, bezogen auf das Gesamtgewicht des Kolostren.

Eine besonders bevorzugte Zusammensetzung des Kolostrumpulvers in der pharmazeutischen Zusammensetzung zeigt Tabelle 2.

In einer bevorzugten Ausführungsform der Erfindung liegt die empfohlene Tagesdosis der pharmazeutischen Zusammensetzung zwischen 5 und 50 g, bevorzugt zwischen 5 und 15 g.

Erfindungsgemäß handelt es sich bei den Erkrankungen, die mittels der pharmazeutischen Zusammensetzung behandelt und/oder die prophylaktisch bekämpft werden sollen und eine gestörte LPS- und/oder Apoptose-Regulation aufweisen um maligne Erkrankungen, insbesondere Tumorerkrankungen (Krebs), nämlich um Tumore des Gastrointestinaltraktes wie bspw. Darmkrebs, Magenkrebs, Pankreaskrebs, besonders bevorzugt um kolorektale Karzinome (CRC).

Als Darmkrebs werden erfindungsgemäß alle bösartigen (malignen) Tumore des Darmes verstanden, vor allem auch die kolorektalen Karzinome (CRC), die mehr als 95 % der bösartigen Darmtumoren ausmachen.

Darmkrebs ist in Deutschland bei Männern und Frauen die zweithäufigste Krebserkrankung (neben Brustkrebs), an der mehr als 6% aller Deutschen im Laufe ihres Lebens erkranken. Kolorektale Karzinome verursachen zunächst sehr selten Symptome, sie entstehen fast immer aus anfangs gutartigen Darmpolypen. Die Heilungschancen durch Operation und Chemotherapie mit 5-Jahres-Überlebensrate von 40 bis 60 % im Mittel hängen entscheidend vom Krankheitsstadium ab, in dem der Darmkrebs entdeckt wird. Die erfindungsgemäße pharmazeutische Zusammensetzung kann, wie mehrmals hingewiesen - nicht nur zur Behandlung von konkret aufgetretenen Erkrankungen, die mit einer gestörten LPS- und/oder Apoptoseregulation assoziiert sind, genutzt werden, vielmehr kann diese auch zur Prophylaxe gegen das Auftreten dieser Erkrankungen eingesetzt werden. Bspw. könnte ein Kolostrum bzw. ein darauf basierendes Präparat und Vitamin D3 in für den regelmäßigen Verzehr geeigneten Mengen zur Verfügung gestellt werden, sodass diese Zusammensetzung prophylaktisch eingenommen werden kann und so die Wahrscheinlichkeit des Auftretens der Erkrankung zu reduzieren hilft.

Die erfindungsgemäße pharmazeutische Zusammensetzung wird in einer weiteren bevorzugten Ausführungsform zur Prophylaxe und/oder zur Behandlung von Erkrankungen mit einer gestörten LPS- und/oder Apoptose-Regulation verwendet. Es handelt sich bei den Erkrankungen um maligne Erkrankungen, insbesondere Tumorerkrankungen (Krebs), bevorzugt um Tumore des Gastrointestinaltraktes wie bspw. Darmkrebs, Magenkrebs, Pankreaskrebs, besonders bevorzugt um kolorektale Karzinome (CRC).

In einer besonders bevorzugten Ausführungsform handelt es sich bei den Erkrankungen um kolorektale Karzinome (CRC) der UICC Stadien I bis IV, bevorzugt der UICC Stadien I bis III, ganz besonders bevorzugt der UICC Stadien I bis II.

Dabei kann unter Behandlung im Falle von malignen Erkrankungen auch die Verminderung von Nebenwirkungen einer weiteren Therapie, z. B. einer Chemotherapie, verstanden werden. Dies beinhaltet auch die Verringerung der perioperativ erhöhten Gefahr einer Tumormetastasierung.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:

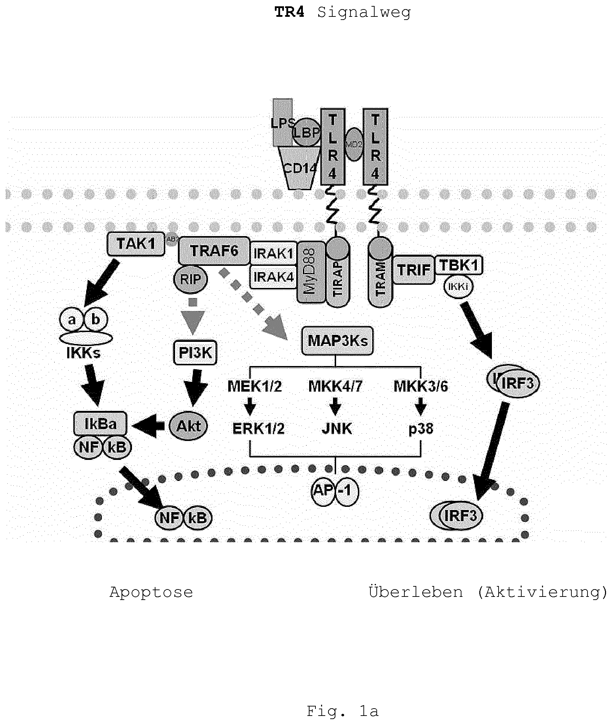

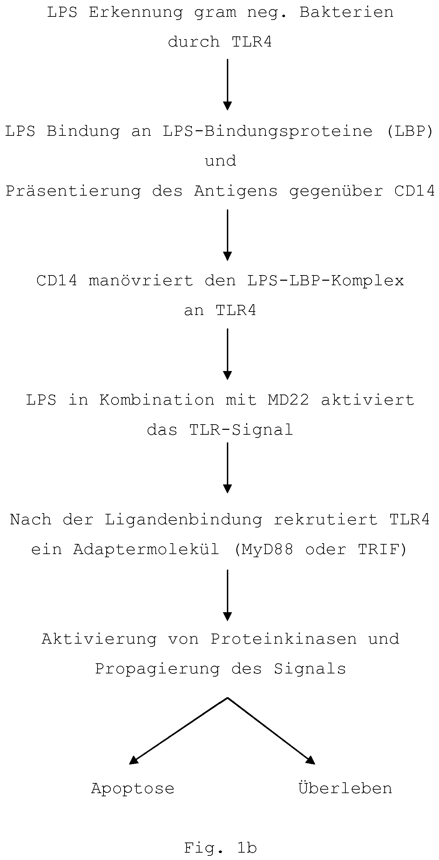

- Fig. 1a,b: eine schematische Darstellung des TLR4-Signalweges als eine repräsentative Zielstruktur der erfindungsgemäßen pharmazeutischen Zusammensetzung

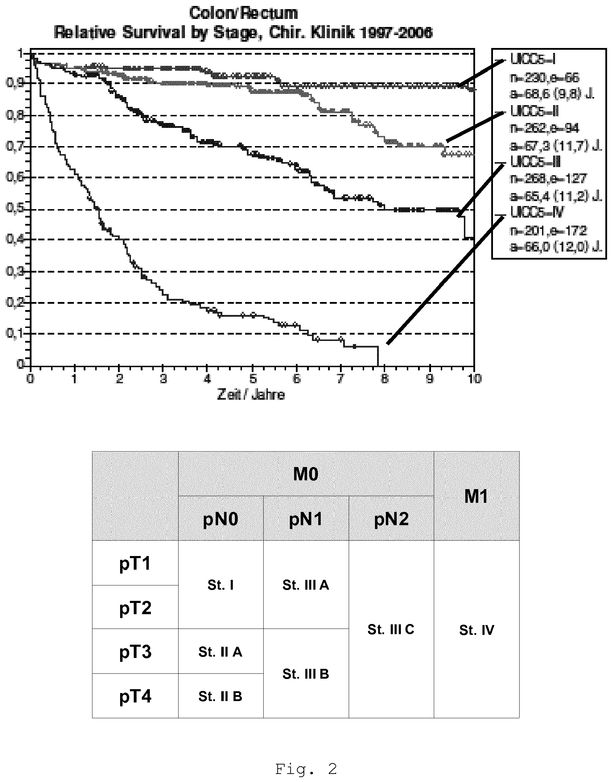

- Fig. 2: eine graphische Darstellung der Gesamtüberlebensraten des Langzeit-Follow-up unterschiedlicher Darmkrebspatienten (Colon-Cancer-Tumor-Development); Gesamtüberleben CRC Interimsanalyse Comprehensive Cancer Center UKW Würzburg, 1997 - 2006, n = 961 Patienten; Die Tabelle zeigt die Klassifikation der einzelnen UICC Stadien nach TNM-System;

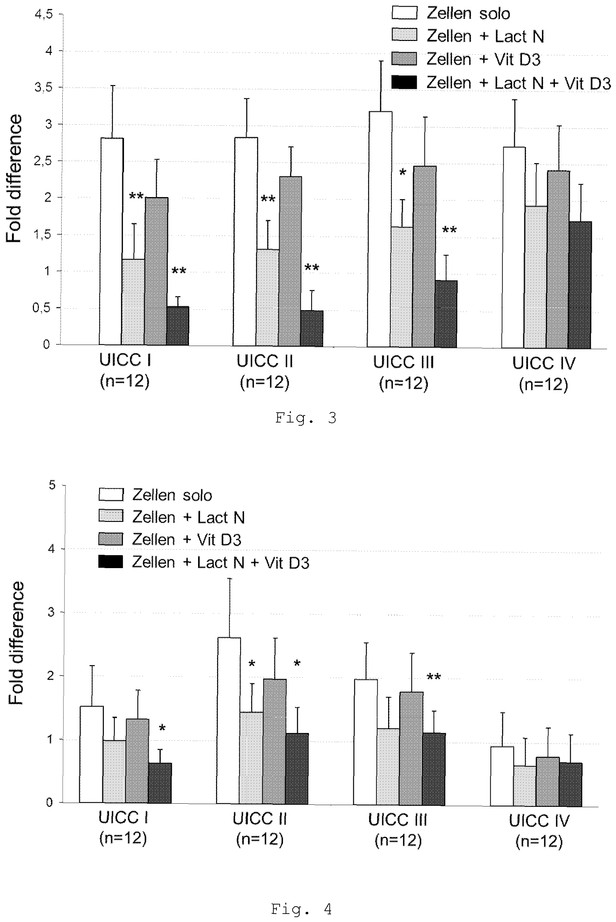

- Fig. 3: ein Säulendiagramm der gemessenen CD68-Genexpression in Monozyten aus dem Blut von Patienten mit CRC der Stadien UICC I bis IV (Messung mit RT-qPCR); * p<0,001; ** p<0,0001 im Vergleich mit Zellen solo; jeweils 12 Proben für jedes Stadium UICC (Säulen von links nach rechts bei jedem Stadium: Zellen solo, Zellen + Lactobin N, Zellen + Vitamin D3, Zellen + Lactobin N + Vitamin D3);
- Fig. 4: ein Säulendiagramm der gemessenen TLR4 Genexpression in Monozyten aus dem Blut von Patienten mit CRC der Stadien UICC I bis IV (Messung mit RT-qPCR); * p<0,001; ** p<0,01 im Vergleich mit Zellen solo (Säulen von links nach rechts bei jedem Stadium: Zellen solo, Zellen + Lactobin N, Zellen + Vitamin D3, Zellen + Lactobin N + Vitamin D3);

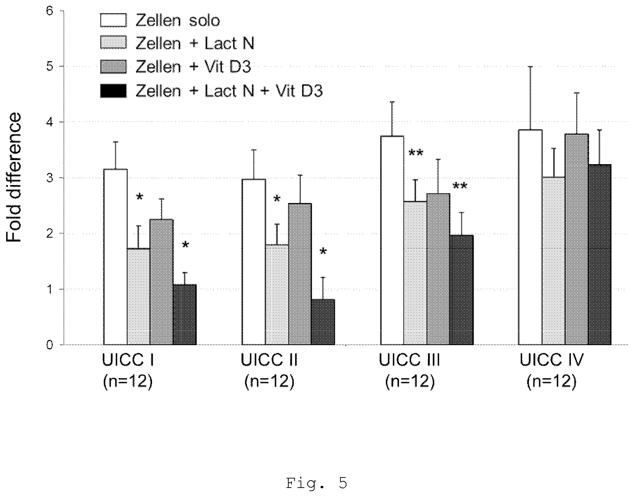

- Fig. 5: ein Säulendiagramm der gemessenen IGF 1-Genexpression in Monozyten aus dem Blut von Patienten mit CRC der Stadien UICC I bis IV (Messung mit RT-qPCR); * p<0,001; ** p=0,02 im Vergleich mit Zellen solo (Säulen von links nach rechts bei jedem Stadium: Zellen solo, Zellen + Lactobin N, Zellen + Vitamin D3, Zellen + Lactobin N + Vitamin D3);

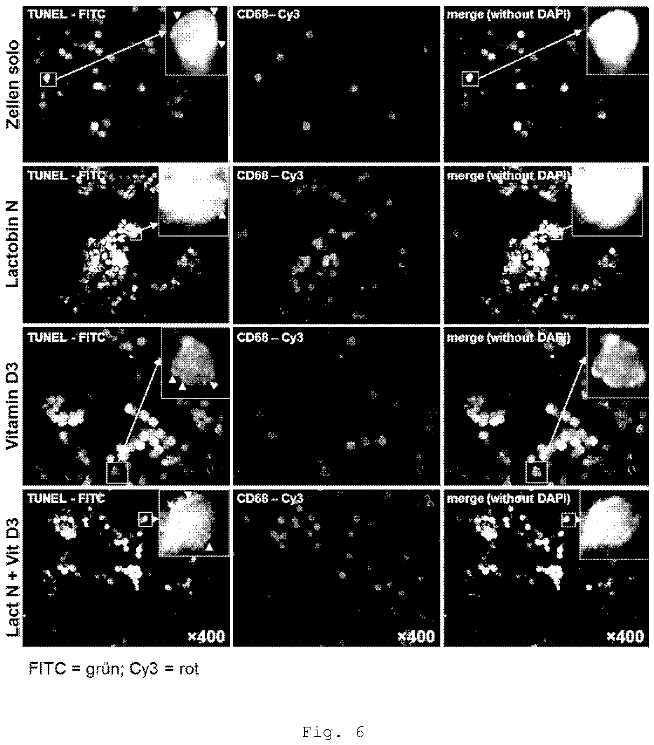

- Fig. 6: Tunel-Färbungen CD68 positiver MNZ aus der Zellkultur und zuvor aus dem Blut isolierter mononukleärer Zellen von Patienten mit CRC (Repräsentatives Beispiel - UICC II)

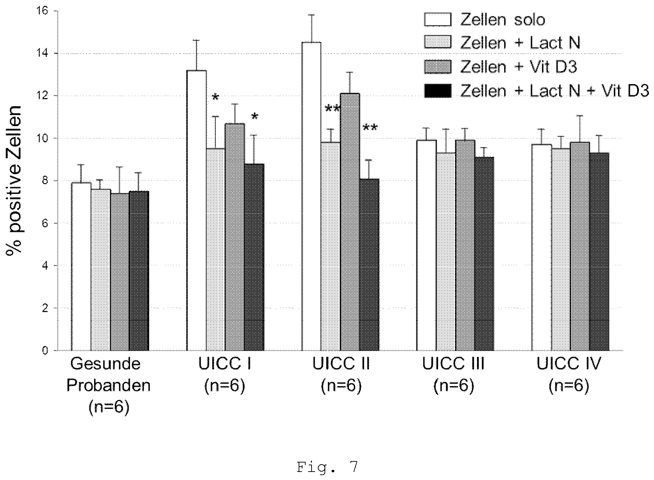

- Fig. 7: ein Säulendiagramm der gemessenen Monozytenzahlen im peripheren Blutbild von Patienten mit CRC der Stadien UICC I bis IV (FACS-Analyse); * p<0,001; ** p<0,0001 im Vergleich mit Zellen solo (Säulen von links nach rechts bei jedem Stadium: Zellen solo, Zellen + Lactobin N, Zellen + Vitamin D3, Zellen + Lactobin N + Vitamin D3);

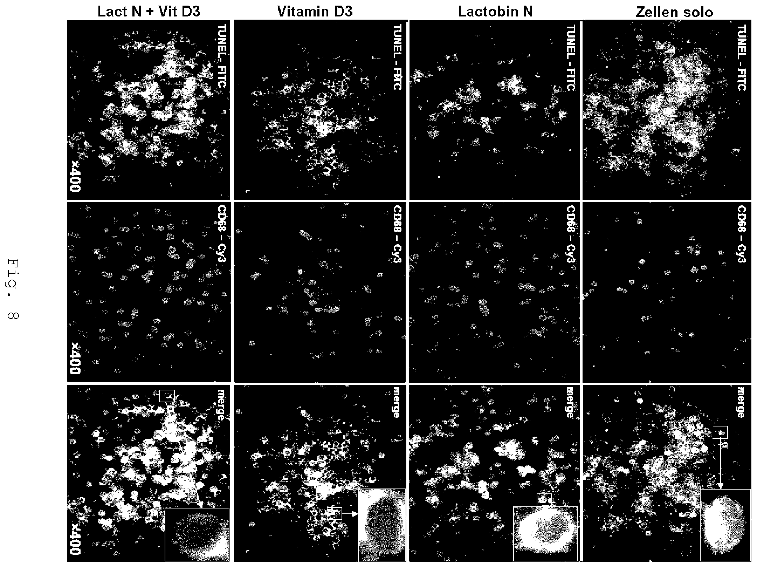

- Fig. 8: Tunel-Färbungen CD68 positiver Makrophagen (CD68) aus Tumorzellen von Patienten mit CRC (Repräsentatives Beispiel für UICC II);

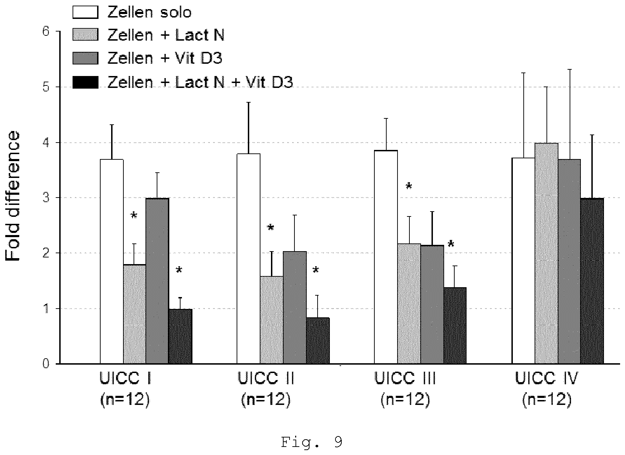

- Fig. 9: ein Säulendiagramm der gemessenen CD68-Genexpression im Tumorkompartiment von Patienten mit CRC der Stadien UICC I bis IV (Messung RT-qPCR); * p<0,0001 im Vergleich mit Zellen solo (Säulen von links nach rechts bei jedem Stadium: Zellen solo, Zellen + Lactobin N, Zellen + Vitamin D3, Zellen + Lactobin N + Vitamin D3) ;

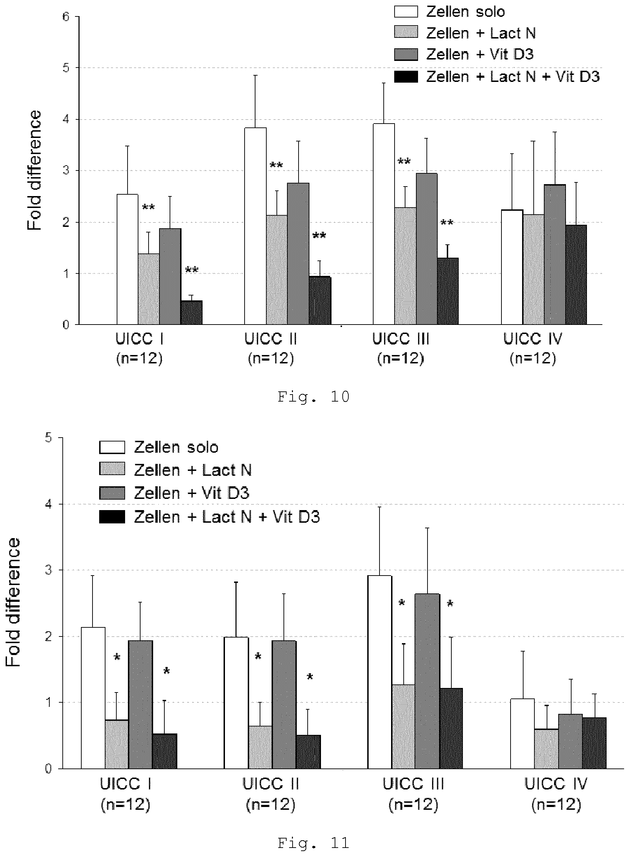

- Fig. 10: ein Säulendiagramm der gemessenen CD14-Genexpression im Tumorkompartiment von Patienten mit CRC der Stadien UICC I bis IV (Messung RT-qPCR); * p<0,0001 im Vergleich mit Zellen solo (Säulen von links nach rechts bei jedem Stadium: Zellen solo, Zellen + Lactobin N, Zellen + Vitamin D3, Zellen + Lactobin N + Vitamin D3) ;
- Fig. 11: ein Säulendiagramm der gemessenen TLR4-Genexpression im Tumorkompartiment von Patienten mit CRC der Stadien UICC I bis IV (Messung RT-qPCR); * p<0,001 im Vergleich mit Zellen solo (Säulen von links nach rechts bei jedem Stadium: Zellen solo, Zellen + Lactobin N, Zellen + Vitamin D3, Zellen + Lactobin N + Vitamin D3) ;

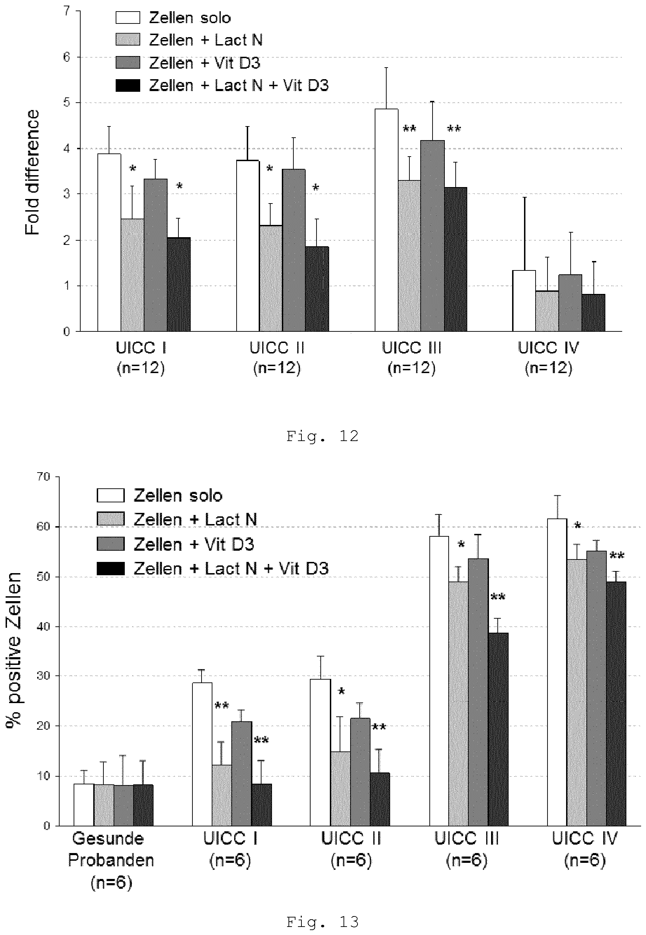

- Fig. 12: ein Säulendiagramm der gemessenen IGF 1-Genexpression im Tumorkompartiment von Patienten mit CRC der Stadien UICC I bis IV (Messung RT-qPCR); * p<0,001; ** p<0,01 im Vergleich mit Zellen solo (Säulen von links nach rechts bei jedem Stadium: Zellen solo, Zellen + Lactobin N, Zellen + Vitamin D3, Zellen + Lactobin N + Vitamin D3);
- Fig. 13: ein Säulendiagramm der gemessenen CD14-Expression in MNZ von Patienten mit CRC der Stadien UICC I bis IV (FACS-Analyse); * p<0,001; ** p<0,0001 im Vergleich mit Zellen solo (Säulen von links nach rechts bei jedem Stadium: Zellen solo, Zellen + Lactobin N, Zellen + Vitamin D3, Zellen + Lactobin N + Vitamin D3);

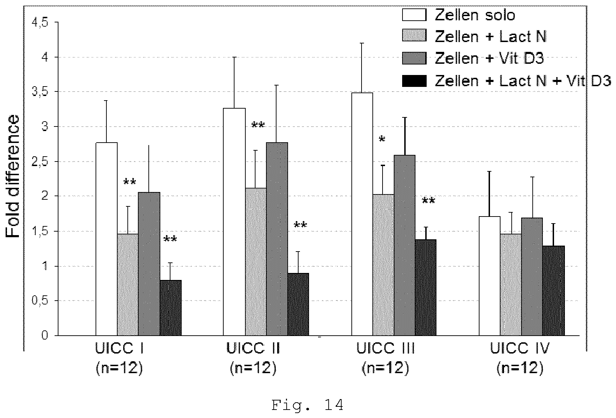

- Fig. 14: ein Säulendiagramm der gemessenen CD14-Genexpression in MNZ von Patienten mit CRC der Stadien UICC I bis IV (Messung RT-qPCR); * p<0,001; ** p<0,0001 im Vergleich mit Zellen solo (Säulen von links nach rechts bei jedem Stadium: Zellen solo, Zellen + Lactobin N, Zellen + Vitamin D3, Zellen + Lactobin N + Vitamin D3);

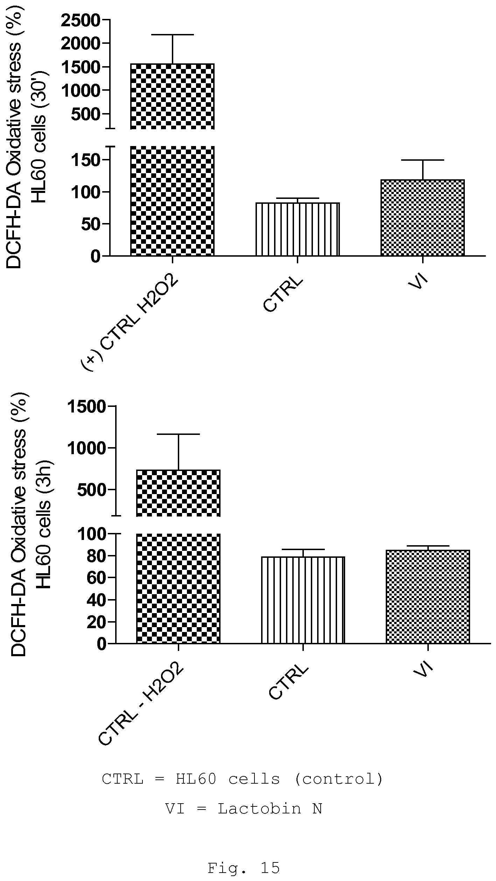

- Fig. 15: ein Balkendiagramm der gemessenen Werte des DCFH-DA (Dichloro-dihydro-fluorescein diacetate) Tests in HL60 Zellen (Human promyelocytic leukemia cells) (oben: 30 min, unten: 3 h);

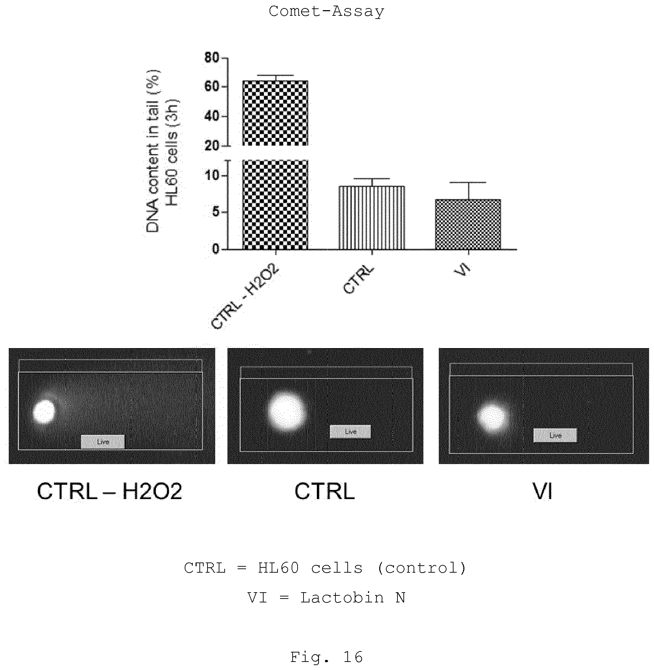

- Fig. 16: ein Balkendiagramm der gemessenen Werte des CometAssays in HL60-Zellen (3 h) und eine graphische Darstellung des Assays;

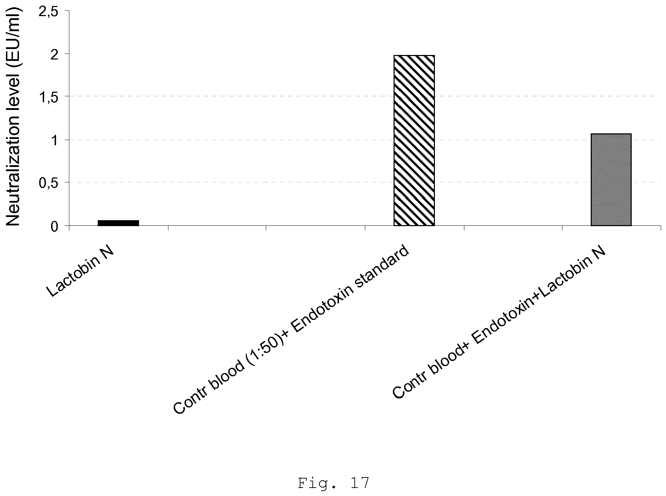

- Fig. 17: Ein Balkendiagramm der gemessenen HintergrundEndotoxinspiegel in Lactobin N sowie die Neutralisationskapazität des Lactobin N;

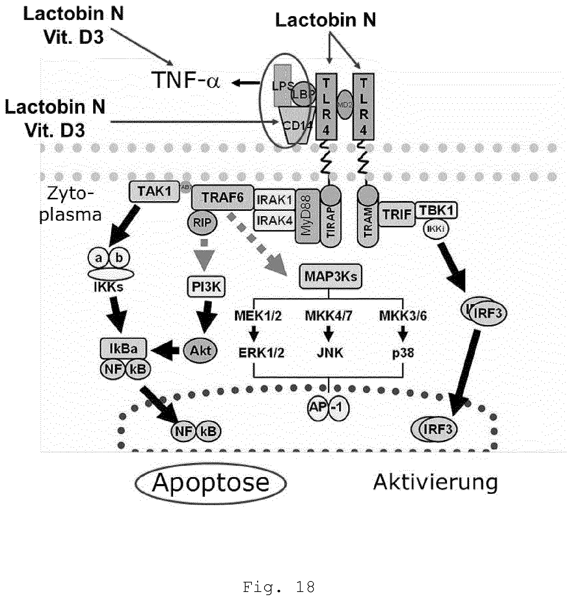

- Fig. 18: eine schematische Darstellung typischer zellulärer Reaktionsmuster im Zuge einer sich formierenden Inflammationsantwort und Wirkmechanismus der erfindungsgemäßen pharmazeutischen Zusammensetzung;

Fig. 1a, b zeigen typische zelluläre Reaktionsmuster, die im Zuge einer sich formierenden Inflammationsantwort hervorgerufen werden. Hierbei spielt insbesondere die Reaktivität des Immunsystems eines Patienten gegenüber LPS, welches aus dem Zerfall gramnegativer Erreger (z. B. *Escherichia coli* und *Enterokokken*) im Blut und auf mukosalen Oberflächen stammt, eine entscheidende Rolle. Auszugsweise wird dies in Fig. 1 dargestellt. Der Ablauf ist in Fig. 1b in einem Flussdiagramm dargestellt.

Fig. 2 zeigt eine graphische Darstellung der Gesamtüberlenbensraten des Langzeit-Follow-up unterschiedlicher Darmkrebspatienten (Colon-Cancer-Tumor-Development; TNM-System: Union International Contre le Cancer, UICC Stadium I-IV).

Fig. 3 zeigt eine erniedrigte CD68-Genexpression in MNZ von Patienten mit CRC unter Inkubation mit dem Kolostrumpräparat Lactobin® N. Weiterhin kann ein zusätzlicher synergistischer Effekt bei der Co-Inkubation von Lactobin® N und Vitamin D3 gezeigt werden.

Fig. 4 zeigt eine erniedrigte TLR4-Genexpression in MNZ von Patienten mit CRC unter Inkubation mit Lactobin® N.

Fig. 5 zeigt eine erniedrigte IGF-1 Genexpression in MNZ von Patienten mit CRC unter Inkubation mit Lactobin® N. Ein zusätzlicher synergistischer Effekt bei Patienten der UICC I-III Klassen bei Co-Inkubation von Lactobin® N und Vitamin D3 kann gezeigt werden.

Fig. 5 zeigt eine erhöhte Apoptoserate CD68 positiver MNZ bei Patienten mit CRC unter Inkubation mit Lactobin® N. Weiterhin kann ein zusätzlicher synergistischer Effekt bei Co-Inkubation von Lactobin® N mit Vitamin D3 (repräsentatives Beispiel, UICC II) gezeigt werden.

Fig. 7 zeigt eine erniedrigte Monozytenzahl im peripheren Blutbild von Patienten mit CRC unter Inkubation mit Lactobin® sowie einen zusätzlichen synergistischen Effekt bei UICC I und II-Patienten bei Co-Inkubation von Lactobin® N und Vitamin D3.

Fig. 8 zeigt eine erhöhte Apoptoserate CD68 positiver Makrophagen aus Tumorzellen bei Patienten mit CRC unter Inkubation mit Lactobin® N. Weiterhin kann ein zusätzlicher synergistischer Effekt bei Co-Inkubation von Lactobin® N mit Vitamin D3 (repräsentatives Beispiel, UICC II) gezeigt werden.

Fig. 9 zeigt eine erniedrigte CD68-Genexpression im Tumorkompartiment von Patienten mit CRC unter Inkubation mit dem Kolostrumpräparat Lactobin® N. Weiterhin kann ein zusätzlicher synergistischer Effekt bei der Co-Inkubation von Lactobin® N und Vitamin D3 gezeigt werden.

Fig. 10 zeigt eine erniedrigte CD14-Genexpression im Tumorkompartiment von Patienten mit CRC unter Inkubation mit dem Kolostrumpräparat Lactobin® N. Weiterhin kann ein zusätzlicher synergistischer Effekt bei der Co-Inkubation von Lactobin® N und Vitamin D3 bei UICC I bis UICC III-Patienten gezeigt werden.

Fig. 11 zeigt eine erniedrigte TLR4-Genexpression im Tumorkompartiment von Patienten mit CRC unter Inkubation mit dem Kolostrumpräparat Lactobin® sowie bei der Co-Inkubation von Lactobin® N und Vitamin D3.

Fig. 12 zeigt eine erniedrigte IGF1-Genexpression im Tumorkompartiment von Patienten mit CRC unter Inkubation mit dem Kolostrumpräparat Lactobin® N. Weiterhin kann ein zusätzlicher synergistischer Effekt bei der Co-Inkubation von Lactobin® N und Vitamin D3 gezeigt werden.

Fig. 13 zeigt eine erniedrigte CD14-Genexpression in MNZ von Patienten mit CRC unter Inkubation mit dem Kolostrumpräparat Lactobin® N. Weiterhin kann ein zusätzlicher synergistischer Effekt bei der Co-Inkubation von Lactobin® N und Vitamin D3 gezeigt werden.

Fig. 14 zeigt ebenfalls eine erniedrigte CD14-Genexpression in MNZ von Patienten mit CRC unter Inkubation mit dem Kolostrumpräparat Lactobin® N. Weiterhin kann ein zusätzlicher synergistischer Effekt bei der Co-Inkubation von Lactobin® N und Vitamin D3 gezeigt werden.

Fig. 15 zeigt keinen signifikant veränderten oxidativen Stress in HL-60 Zellen nach Inkubation mit Lactobin®. Weder nach 30-minütiger noch nach 3-stündiger Inkubation fand sich eine signifikante Verstärkung des oxidativen Stresses.

Fig. 16 zeigt den sogenannten Comet-Assay (auch Einzelzellgelelektrophorese genannt), eine Technik der Gelelektrophorese, durch die es ermöglicht wird, DNA-Schädigungen in einzelnen Zellen festzustellen. Mit diesem Assay können DNA-Einzel- und Doppelstrangbrüche nachgewiesen werden. Das Prinzip des Comet-Assays beruht darauf, dass Zellen in Agarose eingebettet lysiert und einem elektrischen Feld ausgesetzt werden (Elektrophorese). Während der Elektrophorese wandert die negativ geladene DNA zum Pluspol und dank der Poren in der Agarose trennen sich die Bruchstücke der Größe nach auf, da die kleineren Bruchstücke in bestimmter Zeit eine weitere Strecke zurücklegen als die größeren. Chromosomale DNA ist jedoch zu groß, um als Ganzes im elektrischen Feld zu wandern. Nur geschädigte, bruchstückhafte DNA ist hier in der Lage, aus dem Zellkern herauszuwandern. Unter dem UV-Mikroskop erscheinen die beschädigten Zellen, welche vorher mit Fluoreszenzfarbstoffen wie Ethidiumbromid angefärbt wurden, nun mit einem Schweif aus DNA Bruchstücken, der ihnen das Aussehen eines Kometen gibt. Beim Comet-Assay können alle Zellen verwendet werden, die einen Zellkern besitzen. Ausgewertet wird die Zahl der geschädigten und ungeschädigten Zellkerne. In diesem Assay konnte keine signifikante Änderung in der DNA-Schädigung in HL-60-Zellen nach dreistündiger Inkubation mit Lactobin® N beobachtet werden.

Fig. 17 zeigt vernachlässigbare Endotoxinspiegel in Lactobin N (schwarze Säule). Weiter zeigt die Abbildung die Neutralisationskapazität von Lactobin N. Verglichen mit Endotoxin allein als Kontrolle zeigt sich unter Hinzugabe von Lactobin N eine deutliche Neutralisation (ca. 50%).

Fig. 18 zeigt typische zelluläre Reaktionsmuster, die im Zuge einer sich formierenden Inflammationsantwort hervorgerufen werden sowie den Wirkmechanismus der erfindungsgemäßen Zusammensetzung.

Es sind zahlreiche Abwandlungen und Weiterbildungen der beschriebenen Ausführungsbeispiele verwirklichbar.

### Material und Methoden

Das untersuchte Tumorgewebe sowie das periphere Blut stammen von Patienten mit diagnostiziertem Darmkrebs (kolorektales Karzinom; CRC), die in der Tumorgewebe-, Zell- und Serenbank der Universität Würzburg, Chirurgische Klinik und Poliklinik, Zentrum für Operative Medizin, tiefgefroren gelagert wurden. Mononukleäre Zellen aus dem Vollblut und intraoperativ entnommenes Tumorgewebe lieferten die Versuchsergebnisse.

Die peripheren mononukleären Zellen (PBMC: Akronym für engl. Peripheral Blood Mononuclear Cell, d.h. mononukleäre Zellen (MNZ)) des peripheren Blutes. Hierunter werden einkernige Zellen des Blutes bezeichnet, die einen runden Zellkern besitzen. Dies sind zum Beispiel Lymphozyten und Monozyten. Diese Zellen spielen eine wichtige Rolle im Immunsystem bei der Bekämpfung von Infektionen. PBMC bzw. MNZ werden häufig in der Diagnostik als Indikator für vorliegende Infektionen, als Prognosefaktor des Verlaufs einer HIV-Infektion und in der klinischen Forschung genutzt. Aus der Blut- und Serensammlung der Tumorgewebebank und den dort ebenfalls eingelagerten Tumorzellen eines individuellen Tumorpatienten wurden nach Durchlaufen eines spezifischen Auftau-/ Revitalisierungs-Verfahrens auf ihre Protein- und Genexpression, Apoptoseverhalten sowie ihre freigesetzten Botenstoffe (Zytokinprofile) unter Einfluss des Kolostrumpräparates Lactobin® N, Vitamin D3 sowie der Kombination in einem speziell dafür ausgewählten Methoden-Repertoire untersucht.

### Isolierung von Serum und mononukleären Zellen aus dem Patientenblut

EDTA-Röhrchen wurden nach Abnahme 60 min bei Raumtemperatur (RT) stehen gelassen. Danach wurde Serum abgenommen, portioniert (1 ml/Röhrchen) und in -80°C gelagert. Das Blut wurde bei 311g für 10 min zentrifugiert. Danach wurde 1 Teil Vollblut + 5 Teile Lyse Puffer vermischt, 15 min bei RT stehen gelassen und danach bei 311g für 10 min zentrifugiert. Der Überstand wurde verworfen und das Pellet mit RPMI 1640 Medium gewaschen (3x wiederholt). Die Zellen wurden danach gezählt und auf 5x10⁶/ ml in Stickstoff bei -170°C eingefroren (Einfriermedium: RPMI 1640 Medium mit 5% DMSO, Dimethylsulfoxid = Kryokonservierung). Zusätzlich wurde aus einem Röhrchen der Immunstatus gemessen (FACS-Analyse, siehe unten).

### Isolierung von Tumorzellen aus den Kolonkarzinompräparaten

Von jedem Patienten wurde eine Probe des Kolonkarzinoms und des Normalgewebes (des tumortragenden Kolons) oralseitig (proximal, in Richtung oberer Magen-Darmtrakt) entfernt und für Untersuchungen nach folgendem Schema asserviert: Für die molekularbiologische Analyse und immunhistologische Untersuchung wurden die Präparate bis zur Verarbeitung in Gefrierröhrchen und Tissue Tek Röhrchen bei -194°C in flüssigem Stickstoff gelagert; für die RNA Extraktion wurden Gewebeproben mit ca 1,7 ml RNA Stabilisator bei 4°C über Nacht gelagert und später bei -194°C in flüssigem Stickstoff eingefroren; für die Tumorzellanalyse wurden Tumorzellsuspensionen mittels Kollagenasebehandlung und eines Zellsiebes hergestellt und kryokonserviert.

### Auftauvorgang

Das aufzutauende Kryoröhrchen wurde aus dem Stickstoff entnommen und sofort in ein 37°C Wasserbad überführt. Sobald sich die Zellen von der inneren Wand des *Kryotubes* ablösten (1 - 1,5 Min.), wurden sie in ein steriles 50 ml-Falconröhrchen mit 40 ml kaltem Medium überführt. Nach einer 5 minütigen Zentrifugation bei 400g wurde das Medium abgenommen, das Zellpellet in 6 ml angewärmtes Medium resuspendiert und die Zellen wurden gezählt.

### Durchflusszytometrie (Flow cytometry, FACS analysis)

Periphere Blutzellen (MNZs, 5 x 10⁵) wurden mit PE-konjugiertem anti-CD14, FITC-konjugiertem anti-CD45 und 7 AAD, PE-konjugiertem anti-CD25, FITC-konjugiertem anti-CD3 und 7 AAD und PE-konjgiertem anti-Maus IgG2a gefärbt. Alle Antikörper wurden von Beckman Coulter erworben (Krefeld, Germany). Vierfarben-Durchflusszytometrie wurde auf einem FACS-Epics XL-MCL (Beckman Coulter) durchgeführt und die Zellen mit der Expo 32 acquisition software analysiert (Beckman Coulter). Lebensfähige Lymphocyten wurden durch Gating selektiert und 10⁵ Ereignisse aufgezeichnet.

### Protein-Analyse für die Bestimmung von IGF-1

Die Untersuchungen wurden zur Detektion des in den Proben enthaltenen Zytokins Insulin-like growth factor 1 (IGF-1) durchgeführt. Dazu wurden Enzyme-Linked Immunosorbent Assay (ELISA)-Sets (Fa. Biosource International, California, USA) entsprechend den Protokoll-Anleitungen des Herstellers verwendet. In 96-Well Platten, die mit anti-IGF-1 beschichtet waren, wurde pro Well 50 µl Standardprobe, Kontrollansatz und die Proben pipettiert. Danach wurde 50 µl Biotinkonjugat (2. Antikörper gegen IGF-1) hinzugegeben. Anschließend wurden die Platten für 90 min bei Raumtemperatur inkubiert, viermal gewaschen und 100 µl Streptavidin-HRP Working Solution in die Wells pipettiert und die Platte für 45 min bei Raumtemperatur inkubiert. Danach wurden die Platten erneut viermal gewaschen, 100 µl Stabilized Chromogen hinzugeführt und für 30 min bei Raumtemperatur inkubiert. Schließlich wurde 100 µl Stop Solution hinzupipettiert. Die Ergebnisse wurden am ELISA-Reader bei 450 nm abgelesen (Dynatech Laboratories, Sullyfield, USA).

### Protein-Analyse für die Bestimmung von Zytokinen (Luminex-Analyse)

Serum von Vollblutproben wurde durch Zentrifugation erhalten und bei -80 °C bis zur Analyse gelagert. Aliquots des Serums wurden unter Verblindung der Diagnose analysiert.

Interleukin (IL)-12 und proinflammatorische Faktoren wie IL-1β, IL-6, IFN-γ, TNF-α und anti-inflammatorische Faktoren wie IL-10 und IL-13 in den Serumproben wurden als Doppelbestimmung mit einem immunobead-based Multiplex Assay gemessen. Die Assays wurden auf einem Bio-Plex-System durchgeführt (Biosource).

Die mit Gemischen von entsprechenden Fang-Antikörpern beschichteten Mikrosphären und die markierten Detektionsantikörper wurden von Biosource erworben (Camarillo, Germany). Die Reagenzien waren bereits vom Hersteller getestet und qualifiziert worden, um eine Kreuzreaktivität der mit Antikörper beschichteten Mikrosphären auszuschließen.

Die Luminex-Messungen wurden zur Bestimmung weiterer exprimierter Zytokine der MNZ durchgeführt. In 96-Well Platten wurden 25 µl Antikörper-beschichtete Mikrosphären platziert [Gemisch aus den Zytokinen GM-CSF, IFN-γ, IL-1β, IL-2, IL-4, IL-5, IL-6, IL-8, IL-10 und TNF-α; Fertig Kit, Fa. Biosource International, California, USA]. Nach zweimaligem Waschen wurde in jedes Well 50 µl Inkubationspuffer, 50 µl fertige Probenverdünnung und 50 µl Probe pipettiert. Die Platten wurden für 2 Stunden bei Raumtemperatur im Dunkeln auf dem Schüttler inkubiert und erneut zweimal gewaschen. Danach wurde in jedes Well 100 µl des biotinylierten Detektionsantikörpers gegeben, für eine weitere Stunde bei Raumtemperatur im Dunkeln auf dem Schüttler inkubiert, und wieder zweimal gewaschen. In jedes Well wurde 100 µl SAV-RPE (R-Phycoerythrin-Fluoreszenzfarbstoff, Fa. Biosource International) pipettiert, die Platten für 30 min bei Raumtemperatur im Dunklen erneut geschüttelt und dreimal gewaschen. Jeder Testansatz wurde mit 100 µl Waschpuffer pro Well als Endvolumen eingestellt und die Platten im Luminex-Reader (Luminex 100, Fa. Luminex Applied Systems, Sheffield, UK) gemessen.

### Cytospins

Die Zellsuspension aus MNZs und Tumorzellen wurde nach dem Auftauen im RPMI 1640 Medium gewaschen, 10 min bei 311g abzentrifugiert und auf eine Zellzahl von 1x10⁵ Zellen pro ml eingestellt. In jede Cytospinskammer wurden 50 µl Zellsuspension gegeben und 1 min lang zentrifugiert. Die Objektträger wurden unter dem Mikroskop kontrolliert und trockneten 2 Stunden lang bei Raumtemperatur.

Um die Zellen auf Objektträger zu adhärieren, wurden diese in eine Shandon Cytospin 2 Zentrifuge [Thermo Shandon, Pittsburgh, USA] eingespannt. Die Zentrifuge enthält 12 Postitionen zur Befestigung der Objektträger. Zwischen dem Objektträger und dem Einfülltrichter wird ein gelochtes Papier zum Absaugen der überschüssigen Flüssigkeit angebracht. Nach dem Auftauen (-80°C) wurden die Zellen herunterzentrifugiert und zweimal in PBS gewaschen. Anschließend wurde 50 µl der Zellsuspension mit 2x10⁶ Zellen/ml einer jeden Probe in die Trichter gefüllt und die Objektträger für 1 min unter 550 g zentrifugiert. Die Objektträger wurden anschließend über Nacht bei Raumtemperatur luftgetrocknet. Dann wurden sie entsprechend dem Protokoll des TUNEL-Assays bearbeitet.

### In-Situ-Detektion von Apoptose durch TUNEL-Assay

Eine nicht isotopenbasierte in-situ DNA-Enden-Markierungstechnik, unter Verwendung von Digoxigenin-dNTP und einer terminalen Transferase, wurde verwendet, um Zellen mit fragmentierter DNA zu identifizieren (ApopTag® Plus Peroxidase in Situ Apoptosis Kit, Chemicon, Planegg-Muenchen, Germany). Cytospins von peripheren Blutlymphozyten wurden fixiert und die endogene Peroxidase wurde mit 0.1% Hydrogenperoxid gequencht. Die Zellen wurden in dem Puffer der terminalen Transferase äquilibriert, ehe der Reaktionspuffer mit Digoxigenin-dNTP-Oligonukleotid zugegeben wurde. Die mit dem Digoxigenin-dNTP verlängerten Oligonukleotide wurden mit einem Anti-Digoxigenin-Peroxidase-Konjugat detektiert, verdünnt mit einem blocking agent, gefolgt von Färbung (Nova Red, Linaris, Wertheim-Bettingen, Germany) und einem double-stain block (DAKO, Jena, Germany), um eine Kreuzreaktion mit der Oberflächenfärbung zu verhindern. Der CD68 (monocytes) Antikörper (DAKO) wurde über Nacht in einer Feuchtkammer inkubiert. Nach dem Waschen wurden die Cytospins mit einem mit einer Alkaliphosphatase konjugierten anti-Maus-Antikörper (DAKO) inkubiert. Die Cytospins wurden erneut gewaschen und gefärbt (VECTOR Black Substrate Kit, Vector Laboratories, Peterborough, UK) und gegengefärbt (haemalaun staining). Für Negativkontrollen wurden die Cytospins mit der TUNEL-Reaktionsmischung ohne die terminale Transferase (TdT) inkubiert.

TUNEL/CD68 Doppelfärbung wurde durchgeführt, um apoptotische CD68 Zellen im peripheren Blut zu identifizieren und zu zählen. Zunächst wurde TUNEL Färbung durchgeführt wie vorstehend beschrieben, mit Nova Red als Substrat. Danach wurde ein Anti-CD68-Antikörper (DAKO) angewendet, gefolgt von der Inkubation mit VECTOR Black als Substrat. Wenn die behandelten Proben unter dem Lichtmikroskop betrachtet wurden, sind die CD68 positiven Zellen schwarz gefärbt, während die doppelt positiven TUNEL/CD68 positiven Zellen eine rote Färbung des Zellkerns aufweisen.

Der apoptotische Index (AI) wurde definiert als das Verhältnis zwischen TUNEL-positiven infiltrierten Zellen zu allen gezählten infiltrierten Zellen x 100. Für jede Gruppe, die Anzahl an gefärbten Zellen bei einer Vergrößerung von 400 x in mindestens 10 Hauptgesichtsfeldern (400 x) gezählt. Die Zellen wurden als apoptotisch klassifiziert, wenn die gesamte Fläche des Zellkerns als positiv markiert war.

### Genexpressionsanalyse

### RNA Extraktion

Die RNA wurde mit Hilfe des RNA Extraktions Kits (Qiagen, Hilden, Germany) aus MNZs und homogenisiertem Tumorgewebe extrahiert, in DEPC-75%-iger Ethanollösung gewaschen und anschließend bei -70°C bis zur weiteren Verarbeitung gelagert. Die Menge der RNA wurde bestimmt, indem die Absorption bei 260nm gemessen wurde. Die Ratio 260:280 wurde innerhalb des 1,8-2,0 Bereiches festgelegt; eine Kontamination der RNA mit Proteinen konnte damit verhindert werden.

### Herstellung der cDNA (reverse Transkription)

Die komplementäre DNA (cDNA) wurde mittels des iScriptTMcDNA Synthesis Kit (Bio-Rad, München) hergestellt: zu jeweils 45 µl Lösung mit 3 µg jeder RNA im nukleasefreien dest. H₂O, wurden 12 µl des 5x iScript Reaction Mix und 3 µl der iScript Reversen Transcriptase hinzugegeben. Das ergab ein Volumen von 60 µl je Probe. Im iCycler (Bio-Rad, München) erfolgte die reverse Transkription der mRNA zu cDNA nach folgendem Transkriptionsprotokoll: 5 min bei 25°C, 30 min bei 42°C und 5 min bei 85°C. Nach Abschluss der Reaktion wurde die Temperatur auf 4°C gehalten. Die cDNA wurde bei -20°C gelagert.

### Quantitative Real Time PCR

Die Methode der 'Real-time reverse transcription polymerase chain reaction' (kurz: Real-time RT-PCR) wird zur Bestimmung der Expression von verschiedenen Genen eingesetzt. Diese Methode erlaubt die Quantifizierung der Expression eines fraglichen Gens in Relation zu den Kontrollgenen.

Mittels der PCR wurde die in den MNZ und Kolonkarzinomproben exprimierte mRNA von CD14, CD68, TLR4 und IGF-1 analysiert. Die Primer wurden unter Anwendung der Primer Express Software für Primerdesign erstellt, um kurze Amplifikate von 100-200 Basenpaaren der vorgegebenen cDNA zu produzieren. Die quantitative Real Time PCR wurde unter Anwendung von 11,5 µl LightCycler-DNA Master SYBR Green I mix (Applied Biosystems, Darmstadt, Germany) mit 2 µl cDNA (50 ng/µl), 0,3 µM Forward Primer und 0,3 µM Reverse Primer zu einem Gesamtreaktionsvolumen von 23 µl durchgeführt. Der PCR-Zyklus bestand aus fünfzehnminütiger Initialdenaturation bei 95°C, gefolgt von 40 Zyklen fünfzehnsekündiger Denaturationen bei 95°C, einem dreißigsekündigen *Annealing* bei 55-61°C und einer dreisekundigen Extension bei 72°C. Genspezifische Produkte wurden kontinuierlich mit Hilfe des ABI Prism 7700-Sequenzdetektors (Applied Biosystems, Foster City) gemessen und die relative Quantität kalkuliert. Alle Muster wurden in Duplikaten ausgeführt. Eine Quantifizierung während der Datenanalyse erfolgte durch den Farbstoff SYBR Green. Die für die einzelnen Genprodukte gemessenen Amplifikationskurven wurden graphisch dargestellt und in Relation zur Kurve des Kontrollgens ausgewertet. Um die Zielvorlage mengenmäßig zu bestimmen, wurde der durchschnittliche CT-Wert (Schwellenzyklus), also die Anzahl der Zyklen, innerhalb derer die Fluoreszenz des Reporters eine fixierte Schwelle oberhalb der Basiswerte erreicht, bemessen. Dann wurde die Differenz (ΔCT) zwischen den durchschnittlichen CT-Werten der Muster in den vorgegebenen Wells und denen der *housekeeping* Gene, GADPH und β-Actin und dann die Differenz (ΔΔCT) zwischen den durchschnittlichen ΔCT-Werten der Muster für jedes Zielgen und der ΔCT-Wert des Kontrollmusters für dieses Zielgen errechnet. Dieser relative Quantifizierungswert (auch *fold difference*), die Menge, wird als 2^{-ΔΔCT} ausgedrückt.

### Agarosegelelektrophorese

Die Analyse von PCR-Fragmenten erfolgte in einem 2,0%igen (w/v) Agarosegel in einer Horizontalelektrophorese. Je nach Gelgröße und gewünschter Agarosekonzentration wurde die notwendige Menge des Agarosepulvers in einem Erlenmeyerkolben gewogen und das entsprechende Volumen an 1x TBE-Puffer hinzugefügt. Um die Agarose in Lösung zu bringen, musste der Gelansatz in der Mikrowelle mehrmals aufgekocht werden. Zu der heißen Gellösung wurde eine Ethidiumbromidlösung bis auf eine Konzentration von 0,5 mg/ml im Gel zugefügt. Die gewünschte Gelform wurde an den offenen Seiten mit Klebeband verschlossen und je nach gewünschter Trennstrecke ein oder zwei Taschenformer in der Gelform platziert. Mit einer Wasserwaage wurde die Form ausgerichtet. Die etwas erkaltete Agaroselösung wurde in die vorbereitete Gelform gegossen. Das angefertigte Agarosegel wurde in die Elektrophoreseapparatur gelegt. Diese wurde so mit 1x TBE-Puffer gefüllt, bis das Gel komplett bedeckt war. Die zu applizierenden Proben wurden mit 5x Gelbeladungspuffer versetzt, so dass daraus eine einfache Pufferkonzentration resultierte. Durch vorsichtiges Auf- und Abpipettieren wurde die Probe gemixt und in die geformten Taschen des Gels gefüllt. Das aufgetragene Probenvolumen lag bei etwa 13 µl. Zur Bestimmung der Länge der Fragmente wurde ein Längenstandard verwendet. Während der Elektrophorese war anhand der Farbstoffe Bromphenolblau des Gelbeladungspuffers die zurückgelegte Strecke zu beobachten. Die Elektrophorese wurde bei einer Spannung von 90 V für 4 - 5 Stunden durchgeführt.

### Messung des Effekts von Lactobin N auf oxidativen Stress und DNA-Schädigung

Leukozyten sind als Modell zur Bewertung von oxidativem Stress und seiner Modulation von großem Wert. In vitro, hat sich die HL-60 promyelozytische Leukämie-Zellinie als sehr zuverlässig für die Analyse von NADPH Oxidase Aktivität erwiesen. Es lässt sich zunehmend belegen, dass DNA eines der wichtigsten Ziele von oxidativen Angriffen ist. Wenn Reparaturmechanismen die oxidativen DNA-Schäden nicht beheben, kann dies schädliche Folgen für die Zellen haben. Daher wurde der Effekt von Lactobin N auf oxidative DNA-Schädigung und oxidativen Stress in HL-60 Zellen untersucht (Schupp N, Schmid U, Heidland A, Stopper H. Rosuvastatin protects against oxidative stress and DNA damage in vitro via upregulation of glutathione synthesis. Atherosclerosis 2008; 199: 278-287).

### Zellkultur

Die HL-60 (humane promyelozytische Leukämiezellen) Zelllinie (NIH, Washington, USA) wurde in RPMI 1640 ergänzt mit 10% FBS, 1% L-Glutamin, Penicillin (100 units/ml) und 0,1 mg/ml Streptomycin gezüchtet. Die Zellen wurde in einer 6 Well Platte (Sarstedt Inc., Newton, USA) bei einer Anfangskonzentration von 5x10⁵ Zellen/ Well ausgesät, und für 20-24 Stunden nicht behandelt. Dann wurden die Zellen 100 mg/ml Lactobin N und 100 oder 200 µM H₂O₂ für 30 Minutes oder 3 Stunden ausgesetzt. H₂O₂ diente als Kontrolle für oxidative Effekte.

### Flow cytometric analysis of oxidative stress

2,7-Dichlorodihydrofluoreszeindiacetat (H2DCF-DA) wurde verwendet um ROS Produktion in den Zellen zu detektieren. Die HL-60 Zellen wurden vorinkubiert mit 10 µM H2DCF-DA für 15 Minuten bei 37°C, danach wurde die Testsubstanz zugegeben. Die Zellen wurde geerntet und dreimal mit PBS und 1% BSA gewaschen. 5×10⁵ Zellen/Probe wurden mit Durchflusszytometrie mit einem FACS LSR I (Becton-Dickinson, Mountain View, CA, USA) analysiert.

### Comet Assay

Nach Schupp et al. wurde eine alkalische Version des Comet Assays durchgeführt (Schupp N, Schmid U, Heidland A, Stopper H. Rosuvastatin protects against oxidative stress and DNA damage in vitro via upregulation of glutathione synthesis. Atherosclerosis 2008; 199: 278-287). Die Analyse umfasste 50 zufällig ausgewählte Zellen (25 pro Wiederholung (replicate slide)) für jede Probe. Die Proben wurden mit einem Fluoreszenzmikroskop (Labophot 2, Nikon, Germany) bei 200facher Vergrößerung mit Hilfe von Komet 5 image-analysis Software (BFI Optilas, Germany) analysiert. Der prozentuale Anteil von DNA im Schweif (% Tail DNA) wurde zur Quantifizierung der DNA-Wanderung verwendet.

### Endotoxinspiegel und Neutralisationskapazität des Lactobin N

Endotoxine oder Lipopolysaccharide (LPS) sind große (Molekulargewicht: 200 bis 1000 kDa), hitzeresistente (bis zu 100°C) Moleküle, welche die wichtigsten strukturellen Komponenten der äußeren Zellwand von gramnegativen Bakterien bilden. Endotoxin besteht aus einem bioaktiven Lipidteil, genannt Lipid A, kovalent gebunden an ein hydrophobes Heteropolysaccharid mit variabler Länge. Auslösung einer Signaltransduktionskette führt zu der Bindung von Endotoxin an CD14 gefolgt von der Assoziation mit dem Protein MD2 und dem transmembranen Protein TLR4. Dies führt schließlich zu der Freisetzung von entzündlichen Zytokinen, darunter IL-1β, TNF-α and IL-6, hauptsächlich abgegeben von Immunzellen wie Makrophagen und dendritischen Zellen. Zur Zeit ist der LAL-Test der Standardtest zur Bestimmung von Endotoxinen in Arzneimitteln, biologischen Produkten und Medizinprodukten. Allgemein werden drei unterschiedliche LAL-Assays verwendet: Gelierungstest, turbidimetrisch (Zunahme der Trübung) und chromogen (Bildung von Farbe). (Guideline on Validation of the Limulus Amebocyte Lysate Test as an End-Product Endotoxin Test for Human and Animal Parenteral Drugs, Biological Products and Medical Devices. U.S. Dept. of Health & Human Services, FDA, December 1987; Interim Guidance for Human and Veterinary Drug Products and Biologicals. U. S. Department of Health & Human Services, FDA, July 15, 1991; "Bacterial Endotoxins Test." In The U.S. Pharmacopeia, 25th revision, 12601 Twinbrook Parkway, Rockville, MD 20852). Für die Quantifizierung von Endotoxinen wurde der LAL-Test mit kinetischchromogener Detektion verwendet.

### Detektion und Quantifizierung von Endotoxin mit Limulus Amebocyten Lysat (LAL) Endosafe® Endochrome-K™

Gemäß der Richtlinien der U.S. Food and Drug Administration (FDA) wurde der LAL-Test (Charles River Endosafe, Charleston, SC, USA) verwendet und ist für die Quantifizierung von Endotoxinen empfohlen. Es wurde eine Lösung von Lactobin N eingesetzt (Stammlösung 1 mg/ml im Ansatz 1:1 und 1:2 (und 1:4)). Zunächst wurden 0,1 ml von Lactobin N oder 0,1 ml Lactobin N mit Blut von gesunden Freiwilligen in je ein Well einer Mikrotiterplatte pipettiert. Plasma (2 ml) abgeleitet von Heparinblut von Freiwilligen (n=15) wurde zuerst mit 2 ml Endotoxinstandard (50 EU/ml) bei 37°C und 5% CO₂ für 24 Stunden inkubiert. Danach wurden 100 µl weiter mit 100 µl Lactobin N bei 37 °C und 5 % CO₂ für drei weitere Stunden inkubiert. Eine Verdünnung mit H₂O (1:50) wurde durchgeführt und die Lösung durch 5 Minuten bei 75 °C in einem Wasserbad inaktiviert. Die Proben wurden in eine 96-Well-Platte zusammen mit dem Lysat Kit (Limulus amebocyte lysate, Charles River, Charleston, SC, USA) pipettiert und bei 405 nm mit einem Elisa Reader (Endoscan 5, Charles River) vermessen und gemäß der Empfehlungen des Herstellers analysiert (Limulus amebocyte lysate). Um geeignete Verfahrensparameter zu ermitteln wurden den Richtlinien des Herstellers gefolgt. Die Endotoxin-Kontrollproben bestanden aus Endotoxinstandard-Serien aus positiven und negativen Kotrollen. Für die Endotoxinstandard-Serien wurde für jeden Test ein frischer Satz von Verdünnungen aus der Endotoxin-Stammlösung hergestellt. Während des Assays wurde die Erhöhung der Extinktion des Röhrchens oder der Mikrotiterplatte durch ein entsprechendes Messgerät überwacht. Das Messgerät misst die Zeit, welche benötigt wird, bis die Extinktion deutlich über den Hintergrund gestiegen ist, üblicherweise 0,050 bis 0,200 OD Einheiten. Diese Zeit wird als Verzögerungszeit (onset time) bezeichnet. Die Software des Messgeräts erstellt automatisch eine log/log-Korrelation der Verzögerungszeit jedes Standards mit der zugehörigen Endotoxinkonzentration. Die Merkmale der Standardkurve werden angezeigt und evaluiert, um festzustellen, ob die Analyse gültig ist. Die polynomiale Regression wird für den Benutzer mit der Endoscan-V Software berechnet.

Plasma (2 ml) aus Heparinblut von Probanden (n=15) wurde mit 2 ml Endotoxin Standard (50 EU/ml) bei 37°C und 5% CO₂ für 24 Stunden inkubiert. Danach wurde Ansatz 1 (100 µl) mit 100 µl der gelösten Testsubstanzen bei 37°C und 5% CO₂ für weitere 3 Stunden inkubiert. Nach 3 Stunden wurde eine 1:50 Verdünnung mit H₂O hergestellt und für 5 min im Wasserbad bei 75°C inaktiviert. Die Proben wurden zusammen mit Lysat-Kit in eine 96-Well-Platte pipettiert. Die Platte wurde anschließend in einem Elisa-Reader gemessen und ausgewertet.

Es wurden die Asservate von insgesamt 48 Patienten mit histologisch definiertem kolorektalem Karzinom (CRC) untersucht, die in der Chirurgischen Klinik des Universitätsklinikums Würzburg zuvor einer kurativen chirurgischen Resektion unterzogen wurden (TNM-System: Union International Contre le Cancer, UICC Stadium I-IV, vgl. Fig. 2). Die Karzinome stammten von jeweils 12 Patienten pro UICC Stadium, sie wurden hinsichtlich ihrer Lokalisation, ihres Grades an Wandinfiltration (T) und Lymphknotenmetastasierung (N) sowie Vorhandensein einer Fernmetastasierung (M) entsprechend dem gültigen TNM-System und Differenzierungsgrad (Grading, G) kategorisiert und zusammen mit den Patientencharakteristika (Alter und Geschlecht) sowie dem Follow-Up in der Tumordatenbank archiviert. Tumornachsorgeuntersuchungen fanden in regelmäßigen Abständen bei den Patienten gemäß den Richtlinien der Bayerischen Tumorzentren statt. Das vor dem chirurgischen Eingriff entnommene venöse Blut sowie die intraoperativ entnommenen Tumorgewebeproben als auch Kolonnormalgewebe, das während der chirurgischen Resektion nach präoperativer Zustimmung der Patienten angefallen war, wurde asserviert und alle Daten genau dokumentiert. Periphere Blutproben gesunder Individuen (n=12) dienten als Kontrollen.

Die Untersuchungen der Blutmonozyten eines individuellen Patienten sollten der Auffindung von Indikatoren für inflammatorische oder auch etwa therapiebedingte antiphlogistische Abläufe dienen, die durch die Tumorkrankheit bedingt sind, und die gegebenenfalls durch die pharmazeutische Zusammensetzung beeinflusst werden konnten. Die Konzentrationsbereiche, sowohl der Einzelkomponenten der pharmazeutischen Zusammensetzung als auch deren Kombination in den entsprechenden Tumorzellsuspensionen waren approximativ an die bisher bewährten Dosierungen des Kolostrumpräparates Lactobin® in den diversen klinischen Studien, respektive den Einnahmeempfehlungen für Vitamin D3 angepasst worden Die Messwerte wurden dabei nach rechnerischen Verfahren transformiert und in Form von Säulendiagrammen grafisch dargestellt. Für die Datenauswertung wurden der Mann-Whitney U Test und der Student's t Test verwendet. Als statistisch signifikant wurden Werte mit einem P < 0,05 betrachtet.

Das Kolostrumpräparat Lactobin® N stammt von der Firma Dr. Wolz Zell GmbH, Marienthaler Str. 3, 65366 Geisenheim (www.wolz.de). Gemäß den Herstellerangaben handelt es sich hierbei um ein natürliches, ohne Zusatzstoffe aus mindestens 200 Einzelkolostren (Hochpool) hergestelltes Konzentrat (als Pulver) bovinen Ursprungs (Neuseelandrinder), welches wie folgt gewonnen wird:
1. Das Roh-Kolostrum wird von Kühen gewonnen, welche von ihrer Herde abgetrennt wurden, um eine Kontamination mit normaler Milch zu vermeiden;
2. Das Roh-Kolostrum wird gefiltert und bei 4-7 °C gelagert;
3. Verschiedene Roh-Kolostren von unterschiedlichen Höfen werden gesammelt und bei < 12 °C gelagert;
4. Das Fett wird abgetrennt, um die Haltbarkeit des Produkts zu erhöhen. Dies kann mit üblichen Methoden geschehen;
5. Das nun erhaltene Kolostrum wird bei 72 °C für 15 Sekunden pasteurisiert (gemäß EU-Vorgaben);
6. Das Produkt wird durch Membranfiltration und Verdunstung konzentriert bei Temperaturen von < 55 °C;
7. Zur Herstellung eines Pulvers wird bei niedriger Temperatur sprühgetrocknet;
8. Falls notwendig, wird das Kolostrumpulver mit anderen Kolostrumpulvern versetzt, um die in Tabelle 2 angegebenen Inhaltswerte zu erreichen.

Durch diese Vorgehensweise und insbesondere die Verwendung von einem Pool von Kolostren ist sichergestellt, daß das Produkt die geforderte konstante und einheitliche Qualität aufweisen kann. Ein Beispiel für die Werte einer bestimmten Charge des erfindungsgemäßen Kolostrumpulvers zeigt Tabelle 3.

Üblicherweise umfasst das Kolostrumpulver über 80 Gew.% Proteine, <2 Gew.% Fett, <11 Gew.% Laktose, <5 Gew.% Wasser, <0,2 µg/100g Vitamin D (Durchschnittswerte). Es enthält neben Zytokinen, Wachstumsfaktoren und Enzymen auch Immunglobuline (insbesondere der Klasse IgG1). Quantitative Messwerte liegen für IgG (Subklasse IgG1) mit 21-24 Gew.% vor.

Das erfindungsgemäße Kolostrumpulver enthält keine Konservierungsmittel oder Antioxidantien etc.. Es ist dennoch bei ordnungsgemäßer Lagerung mindestens 24 Monate haltbar.

Diesem Kolostrumpulver wird nun erfindungsgemäß Vitamin D3 zugesetzt. Im vorliegenden Fall wird ein mit Maisstärke stabilisiertes Vitamin D3-Präparat zugegeben. Dazu wird Vitamin D3 (Cholecalciferol) und DL-α-Tocopherol in geschmolzenem und teilweise gehärteten Sojaöl gelöst und mit einer wässrigen Lösung von Gelatine und Saccharose zu einer Emulsion verarbeitet. Diese Emulsion wird in Maisstärke gesprüht. Das erhaltene Pulver wird von der überschüssigen Stärke abgetrennt, getrocknet und verpackt. Das erhaltene Pulver enthält Vitamin D3 2,5 mg/g; DL-alpha-Tocopherol 2 mg/g; Teilweise gehärtetes Sojaöl 75 mg/g; hydrolysiertes tierisches Eiweiß 380 mg/g; Saccharose 380 mg/g; Maisstärke 160,5 mg/g.

Zur Herstellung eines erfindungsgemäßen Präparats wurde das vorstehend beschriebene Kolostrumpulver (bevorzugt Lactobin N) mit einer entsprechenden Menge des Vitamin D3 enthaltenden Pulvers versetzt, so dass die erfindungsgemäße Konzentration an Vitamin D3 erreicht wurde. Ein erfindungsgemäß eingesetztes Mischungsverhältnis betrug 0,2 mg des Vitamin D3 enthaltenden Pulvers pro Gramm Kolostrumpulver (0,2 mg/g). Dies ergab für die Proben 0,5 µg Vitamin D3/g Fertigpräparat. Eine empfohlene Tagesdosis beträgt 10 g Fertigpräparat, d.h. 5 µg Vitamin D3.

Das Fertigpräparat (aus Lactobin N) wurde in den Experimenten eingesetzt, wenn eine Kombination von Lactobin N und Vitamin + D3 angegeben wird (z.B. "Lact. + Vit. D3" in Tabelle 1 oder "Lact N + Vit D3 in den Figuren). Ebenso wurde nur das Vitamin D3-Präparat eingesetzt, wenn die Proben nur mit Vitamin D3 behandelt wurden (z.B. "Vit. D3" in Tabelle 1). Kontrollen ohne Vitamin D3 wurden mit Lactobin N (auch als "Lact N" abgekürzt) behandelt.

### Ergebnisse:

Wirkungen der Einzelkomponenten und der Kombination auf MNZ und Tumorzellen von Patienten mit CRC:
Monozyten spielen eine wesentliche Rolle als Ziel- und Effektorzellen der Antigenerkennung und Infektabwehr. Auf ihnen befinden sich definierte Muster an funktionell wichtigen Oberflächenantigenen. Werden Monozyten aktiviert, so kann sich das Muster an Oberflächenantigenen ändern. Die stark erhöhte Gesamtzellzahl an Monozyten in Patienten früher als auch fortgeschrittener Tumorstadien (besonders UICC Stadium III) normalisierte sich unter in vitro-Inkubation mit dem Kolostrumpräparat Lactobin® N. Unter Co-Inkubation mit Vitamin D3 war sowohl bei Monozyten aus UICC Stadium I Patienten als auch UICC Stadium II Patienten ein signifikanter synergistischer Effekt festzustellen (FACS-Analyse): Ein gleichsinniges Verhalten der CD14-positiven MNZ aus UICC Stadium I, -II und -III Patienten nach Inkubation mit Einzelkomponenten und der Kombination (signifikant erniedrigt im Sinne eines synergistischen Effekts) konnte beobachtet werden.

Analog: Erniedrigte CD68-Genexpression in Monozyten aus dem Blut von Patienten der Stadien UICC I bis IV (Messung mit RT-qPCR) sowohl unter den Einzelkomponenten als auch unter der Kombination (Lactobin ® N vs. unbehandelte Kontrolle: UICC I und II: p<0,0001; UICC III: p<0,001 und Lactobin N + Vitamin D3 vs. unbehandelte Kontrolle: UICC I-III: p<0,0001). Der Vergleich der Einzelkomponenten mit der Kombination wies einen Synergismus auf (p<0,01); im Vergleich zu den Einzelkomponenten war die Kombination wiederum statistisch signifikant effektiver (vgl. Fig. 3).

Als entscheidender weiterer Hinweis für eine normalisierte inflammatorische Antwort im Blut von Tumorpatienten mit CRC wurde dazu eine Abnahme der TLR4 Genexpression in den Zellkulturansätzen unter den o.g. Co-Inkubationen in allen Tumorstadien (UICC Stadium I-IV) festgestellt (UICC I-II p<0,001, UICC III p<0,01) (wiederum im Sinne eines Synergismus im Vergleich zu den Einzelkomponenten) (vgl. Fig. 4).

Ausgeprägt zeigte sich ferner der Abfall des Genexpressionsprofils für IGF 1 (Apoptoseinhibitor) im MNZ des Blutes nach 12-stündiger Inkubation mit Lactobin ® N, Vitamin D3 und der Kombination (Lactobin ® N vs. unbehandelte Kontrolle: UICC I und II: p<0,001; UICC III: p=0,02 und Lactobin ® N + Vitamin D3 vs. unbehandelte Kontrolle: UICC I und II: p<0,001; UICC III: p=0,02). Der Vergleich der Einzelkomponenten mit der Kombination wies einen Synergismus in Patienten der Stadien UICC I-III auf (p<0,01) (vgl. Fig. 5).

Die vorhergehend dargestellten Ergebnisse waren Anlass Tunel-Färbungen CD68 positiver MNZ aus der Zellkultur und zuvor aus dem Blut isolierter mononukleärer Zellen von Patienten mit CRC durchzuführen. Sie zeigen eindrücklich verstärkt apoptotische MNZ aus dem Blut der CRC-Patienten (vgl. Fig. 6, repräsentatives Beispiel aus UICC II-Patienten). Die mittels Genexpressionsanalyse weiter oben beschriebenen Befunde wurden für untersuchte Zellen aus Patienten der UICC Stadien I und II mittels FACS bestätigt. Erniedrigte MNZ in FACS-Analyse unter Lactobin® N allein sowie unter der Kombinationsbehandlung (vgl. Fig. 7).

An die Untersuchungen der peripheren Blutmonozyten schloss sich die Analyse des Tumorgewebes an, bestehend aus desintegrierten Zellen (Gesamtfraktion) aus den Tumorgeweben (Tumorzellen und tumorinfiltrierende Makrophagen sowie weitere tumorinfiltrierende Immunzellen). Vergleichbar verstärkte Apoptoseraten tumorinfiltrierender Makrophagen wie die bereits in den periphervenös entnommenen MNZ zeigten sich in den untersuchten Zellen aus den Primärtumorgeweben (vgl. Fig. 8, repräsentatives Beispiel für UICC II).

Die Genexpression für CD68 war in allen Tumorgeweben (UICC Stadium I-IV) unter Inkubation mit Lactobin® N erniedrigt (Lactobin® N vs. unbehandelte Kontrolle: UICC I und II p<0,0001 und Lactobin N + Vitamin D3 vs. unbehandelte Kontrolle: UICC I-III p<0,0001). Die Co-Inkubation mit Vitamin D3 ergab damit synergistische Effekte (UICC Stadium I-III) (vgl. Fig. 9).

Parallel zur erniedrigten CD68 Expression der tumorständigen Makrophagen wurde auch ein Abfall in der Genexpression des Makrophagen-Rezeptors CD14 gemessen (Lactobin® N vs. unbehandelte Kontrolle: UICC I-III p<0,0001 und Lactobin® N + Vitamin D3 vs. unbehandelte Kontrolle: UICC I-III p<0,0001). Die Hinzugabe von Vitamin D3 führte damit zu synergistischen Effekten (UICC Stadium I-III) (vgl. Fig. 10).

Die Genexpression für TLR4 war unter Lactobin® N parallel zur CD14 Expression erniedrigt (Lactobin® N vs. unbehandelte Kontrolle: UICC I-III p<0,001). Die Hinzugabe von Vitamin D3 zeigte keine zusätzlichen Effekte (UICC I-IV) (vgl. Fig. 11).

Für die IGF1-Expression fand sich parallel zu den vorgenannten Genexpressionen in allen Tumorgeweben (UICC Stadium I-IV) ein durchweg erniedrigtes Expressionsprofil unter Lactobin® N (Lactobin® N vs. unbehandelte Kontrolle: UICC I-II p<0,001; UICC III p<0,01). Die Hinzugabe von Vitamin D3 zeigte zusätzlich geringe synergistische Effekte (Lactobin® N + Vitamin D3 vs. unbehandelte Kontrolle) (vgl. Fig. 12).

Eine erniedrigte CD14-Genexpression in MNZ von Patienten mit CRC unter Inkubation mit dem Kolostrumpräparat Lactobin® N konnte in allen UICC Stadien (I-IV) ermittelt werden. Weiterhin konnte ein zusätzlicher synergistischer Effekt bei der Co-Inkubation von Lactobin® N und Vitamin D3 gezeigt werden (vgl. Fig. 13 und Fig. 14; Messungen mittels RT-qPCR und FACS-Analyse).

Der Test auf oxidativen Stress in HL-60 Zellen wurde zum Ausschluss von negativen Effekten bei direkter Inkubation monozytärer Zellen mit Lactobin® N durchgeführt. Weder nach 30-minütiger noch nach 60-minütiger Inkubation fand sich eine signifikante Verstärkung des oxidativen Stresses (vgl. Fig. 15).

Im Comet-Assay wurde parallel zum vorgenannten Test auf oxidativen Stress keine signifikante Änderung in der DNA-Schädigung in HL-60-Zellen nach dreistündiger Inkubation mit Lactobin® N beobachtet (vgl. Fig. 16).

Im LPS-Neutralisationstest wurde eine deutliche Neutralisationskapazität für Lactobin N beobachtet(vgl. Fig. 17).

Im Luminex-Assay wurden weitere direkte Hinweise für eine herunterregulierte inflammatorische Antwort (IL-1β, IL-6, IFN-γ und TNF-α) in den MNZ aus Tumorpatienten mit CRC parallel zu den herunterregulierten Genexpressionen für CD68, CD14 und TLR4 in den Zellkulturansätzen unter den o.g. (Co-)Inkubationen in allen Tumorstadien (UICC Stadium I-IV) festgestellt (unter Co-Inkubation: UICC Stadium I und II p<0,01) (Tabelle 1).

### Diskussion / Schlussfolgerungen

Aus Untersuchungen von Patienten mit soliden Tumoren gibt es zunehmend Hinweise für verstärkte inflammatorische Reaktionen bei fortschreitender Tumorerkrankung. Ursache und Auswirkungen dieser auf erhöhtem Grundniveau ablaufenden inflammatorischen Immunantworten sind bis heute nur zum Teil geklärt. Häufig zu beobachten ist trotz verstärkt messbarer Inflammationsparameter im Blut eine klinische Schwächung des Tumorpatienten in seiner Bereitschaft auf infektiologische Probleme und auf sein Tumorgeschehen in Abhängigkeit seines Fortschreitens zu reagieren. Daneben existieren mittlerweile mehrere Arbeiten darüber, wie das Immunsystem eines Tumorpatienten auf zellulärer und molekularer Ebene auf ein Tumorgeschehen antwortet (spezifische Tumorimmunantwort, Effektorzellantwort, spezifische T-Zellzytotoxizität, Antikörper-vermittelte Tumorabwehr). Prognostische Vorhersagen wurden bisher mehr aus der Infiltrationsdichte bestimmter Immunabwehrzellen in einem soliden Tumor abgeleitet, die in sich durchaus schlüssig erscheinen. Vor dem Hintergrund, dass ein solider Tumor wie z. B. ein kolorektales Karzinom (CRC) bei fortschreitendem Tumorwachstum verstärkt ein inflammatorisches Mikromilieu in sich trägt (inflammatorische Zellen und Botenstoffe) und inflammatorische Parameter zunehmend auch im peripheren Blut messbar werden, ist für die Behandlung von Tumorerkrankungen eine notwendige therapeutischen Normalisierung und damit Modulation dieser übersteuerten Inflammationsantwort zwingend. Dieses erfolgt aus der Erkenntnis heraus, dass eine chronisch verstärkte inflammatorische Grundaktivität die Fähigkeit zur immunologischen Auseinandersetzung mit einem Tumorgeschehen und vor allem dessen spezifischen Abwehr herabsetzt.

Abhängige Faktoren einer immunologischen Auseinandersetzung sowie insbesondere Indikatoren für inflammatorische Reaktionsabläufe, die durch Tumorerkrankungen bedingt sind, können durch die erfindungsgemäße pharmazeutische Zusammensetzung bspw. bei Patienten mit CRC, beeinflusst werden und wurden daher in Tumorzellen der betroffenen Patienten untersucht. In diesen Patienten zeigte sich eine verstärkte Reaktivität gegenüber Lipopolysacchariden (LPS) und das Vorliegen einer überschießenden inflammatorischen Reaktionsbereitschaft.

Die Reaktivität des Immunsystems eines Patienten gegenüber LPS, welches aus dem Zerfall gramnegativer Erreger (z. B. *Escherichia coli* und *Enterokokken*) im Blut und vor allem auf mukosalen Oberflächen stammt, ruft typische zelluläre Reaktionsmuster im Zuge einer sich formierenden Inflammationsantwort hervor, wie dies auszugsweise in Fig. 1 dargestellt ist. In Fig. 18 wird der Wirkmechanismus der erfindungsgemäßen pharmazeutischen Zusammensetzung wiedergegeben.

Die verstärkte Triggerung einer inflammatorischen Antwort über den TLR4-Signalweg stellt somit ein im klinischen Alltag häufig anzutreffendes Reaktionsmuster dieser Patienten dar. TLR4, ein in der Evolution hochkonserviertes Element der Inflammationsantwort, ist hierfür ein idealer Vertreter und wurde daher für die Messung inflammatorischer Geschehen in einem Tumorpatienten gezielt und erstmalig ausgewählt.

TLR4 wird (unter anderem) von Monozyten und Makrophagen exprimiert. Der Nachweis dieses Rezeptors, dort insbesondere in Zusammenhang mit den entsprechenden Zellzahlen, ist mithin kennzeichnend für eine aktuelle oder bereits durchgemachte Inflammationsantwort auf bakterielle Erreger in einem Tumorpatienten, wobei dies - eine in der Regel überschießende Reaktion - für den Patienten von großer pathophysiologischer Bedeutung ist. Seine - wie in dieser Ausprägung erstmalig erfindungsgemäß gezeigt - übersteuerte Reaktion auf aus dem Magen-Darmtrakt einströmendes LPS stellt dabei einen realistischen Krankheitswert dar.

Monozyten / Makrophagen gehen nur unter bestimmter Signalwirkung, das heißt "unter Aufforderung" in den "selbst-induzierten Zelltod". Die Schwelle zur Apoptoseinduktion in den Zellen bei Tumorpatienten ist - wie erfindungsgemäß gezeigt werden konnte - verschoben, da messbar überhöhte Monozyten-Zellzahlen gerade in Patienten mit zunehmender Tumorlast vorliegen. Dieses Phänomen konnte beobachtet werden, ohne dass gleichzeitig klinisch konkret erkennbare Zeichen einer bakteriellen oder viralen Infektionskrankheit feststellbar waren. Dies zeigt, dass im Zuge der Tumorprogression und der zunehmenden Inflammationsantwort erhöhte Schwellenwerte zur Auslösung von Apoptose in den Monozyten vorhanden waren.

Lactobin® N und Vitamin D3 sind seit Längerem in medizinischem Gebrauch. Unter den zahlreichen auf dem Markt befindlichen Kolostrumpräparaten verfügt Lactobin® N über den wohl breitesten Sockel an präklinischen und klinischen Daten. Es zeichnet sich durch die hohe Gleichförmigkeit seiner Produktionschargen aus, was für ein solches hochkomplexes Wirkstoffgemisch von größter Wichtigkeit ist. Ein Befund stammt dabei aus einer klinischen Studie mit 40 bauchchirurgischen Patienten, die perioperativ mit Lactobin® oder mit Placebo behandelt wurden. Dabei wiesen die mit dem Kolostrumpräparat behandelten Patienten einen statistisch signifikant niedrigeren LPS-Spiegel in ihrem Serum auf, was auf dessen intraenterale Neutralisation schließen lässt (Edwin Bölke et al.: PREOPERATIVE ORAL APPLICATION OF IMMUNOGLOBULIN-ENRICHED COLOSTRUM MILK AND MEDIATOR RESPONSE DURING ABDOMINAL SURGERY. SHOCK, Vol. 17, No. 1, pp. 9-12, 2002). In dieser Hinsicht ist die Polyklonalität der anti-LPS Spezifitäten (Anti-O-Kettendiversität) entscheidend.

Letztendlich ist die Anti-O-Kettendiversität auch dafür verantwortlich, dass die Toxin-Neutralisation keiner Resistenzentwicklung unterliegt. Die Kolostrumproduktion während der ersten postpartalen Tage passt sich ununterbrochener Immunisierung des Muttertieres fortlaufend dem Antigenspektrum seiner Umgebung an, was durch die Wanderung der Herdentiere einerseits und durch das Poolen im Durchschnitt von mehr als 200 Einzelkolostren für die Produktion einer Lactobin-Charge noch weiterhin zu einer Diversitätsverstärkung beiträgt. Des Weiteren wird durch die anti-Lipid-A-Neutralisationskapazität über das im Lactobin® ebenfalls vorhandene Lactoferrin das LPS Neutralisationspotential zusätzlich verstärkt. Vor diesem Gesamthintergrund erscheint die Durchführung der schon erwähnten Pharmakodynamik-Studie von Waaga-Gasser et al. (A.M. Waaga-Gasser et al.: Oral immunoglobulin induces mononuclear cell apoptosis in patients suffering from idiopathic chronic pain syndrome: Results from a pilot study. International Journal of Clinical Pharmacology and Therapeutics, Vol. 47 - No. 7/2009, pp. 421-433) plausibel, in welcher vor allem mittels der TUNEL-Technik die Induktion der Wiederherstellung normaler Apoptoseverhältnisse durch Lactobin® N parallel zur Besserung der klinischen Symptomatik der behandelten Schmerzpatienten gezeigt werden konnte.

Vitamin D weist in vitro eine Vielzahl von Effekten auf, die auf eine immunmodulatorische Wirkung hinweisen. Von besonderem Interesse ist, dass im klinischen Versuch keine immunsuppressive Wirkung nachgewiesen werden konnte. In einer klinischen Studie an Patienten mit CRC war eine Abhängigkeit der 25(OH)D-Serumspiegel nachgewiesen worden (Stubbins RE et al.: 2012. Using components of the Vitamin D pathway to prevent and treat colon cancer. Nutrition Reviews 70(12): 721-729).

Die erfindungsgemäße pharmazeutische Zusammensetzung bewirkt bei oraler Einnahme weitere pharmakodynamische Prozesse an der Darmmukosagrenze mittels des dort ortsständigen MALT-Systems (Mucosa Associated Lymphatic Tissue). In diesem lokalen Teilsegment des Immunsystems spielt sich die Auseinandersetzung mit Umweltpathogenen ab, wobei hieraus resultierende überschießende Reaktionen aktivierter peripherer Monozyten über eine präparatbedingte Apoptoseinduktion auf Normalmaß zurückgeführt werden. In Tumorpatienten wird demnach schon lokal die inflammatorische Reaktionsbereitschaft reguliert, was sich in normalisierten Monozytenzahlen, normalisierter CD14- und TLR4 Expression und den zurückgeführten IGF1 Profilen ausdrückt.

Die über den Gastrointestinaltrakt hinausgehende Wirkung von Lactobin® N auf infiltrierte immunkompetente Zellen im Tumorgewebe ist, verglichen mit periphervenösen Monozyten, in allen Tumorstadien entsprechend gefunden worden, was zeigt, dass das übersteuerte inflammatorische Mikromilieu ausgehend von monozytären Zellen, die in das Tumorgewebe infiltriert waren, durch Gabe von Lactobin® und Vitamin D3 herunterreguliert werden kann.

Das zeigt eindeutig, dass bei normalisierter systemisch messbarer Inflammationsantwort und unterdrücktem inflammatorischen Mikromilieu im Tumorgewebe immunologische Tumorabwehrmechanismen deutlich effektiver ablaufen können.

Die vorliegenden Untersuchungsergebnisse stellen eine ungewöhnlich solide Betrachtung der Reaktionsabläufe bei Tumorpatienten, insbesondere Darmkrebspatienten (CRC) dar. Dies zeigen die Ergebnisse der hier berichteten Wirksamkeitsuntersuchung mit Lactobin® N / Vitamin D3 von menschlichen Tumor- und blutständigen Entzündungszellen. Es sind besonders die synergistischen Effekte bei kombiniertem Einsatz für Patienten mit kolorektalen Karzinom bedeutungsvoll, nicht nur für eine Primärprävention, sondern auch für eine Sekundärprävention nach radikaler Tumoroperation zur Steigerung der T-Effektorzell-Antwort bei okkulten Fernmetastasen (Minimal Residual Disease nach erfolgter radikaler Tumorresektion).

Die beschriebene Gleichförmigkeit der Produktion von Lactobin® und die daraus resultierende Homogenität der Produktionschargen ist von grundsätzlicher Bedeutung für die Validität der analytischen, präklinischen und klinischen Daten zu dem Produkt.

| **Tabelle 1. Zytokinproduktion von MNZ nach Inkubation mit Lactobin® N, Vitamin D3, Lactobin ® N + Vit D3** | | | | | | | | | |
|---|---|---|---|---|---|---|---|---|---|
| | | **IL-1β** pg/ml | **IL-6** pg/ml | **IFN-γ** Pg/ml | **TNF-α** pg/ml | **IL-12** pg/ml | **IL-10** pg/ml | **IL-13** pg/ml | **IGF-1** ng/ml |
| **Kontrolle (n=6)** | Zellen ohne Zusatz | 4,7 ±0,4 | 3,1 ±0,6 | 12,6 ±2,3 | 62,1 ±10,3 | 47,8 ±6,5 | 27,5 ±4,2 | 43,3 ±9,3 | 93,4 ±6,3 |
| | Lact N | 4,5 ±0,6 | 3,0 ±0,2 | 12,3 ±3,1 | 62,2 14,2 | 49,1 ±7,1 | 27,8 ±4,7 | 44,1 ±11,2 | 91,4 ±5,4 |
| | Vit D3 | 4,8 ±0,1 | 3,3 ±0,3 | 12,8 ±4,7 | 62,6 ±10,7 | 47,7 ±8,3 | 27,9 ±5,8 | 43,6 ±10,6 | 90,3 ±4,7 |
| | Lact + Vit D3 | 4,5 ±0,4 | 3,0 ±0,1 | 12,1 ±5,2 | 62,2 ±10,3 | 41,1 ±6,9 | 28,1 ±4,8 | 44,9 ±9,5 | 89,1 ±4,1 |
| **UICC I (n=6)** | ohne Zusatz | 60,1 ±7,8 | 99,2 ±10,8 | 71,8 ±6,3 | 120,1 ±6,5 | 97,6 ±8,3 | 11,9 ±6,4 | 23,0 ±7,3 | 195,2 ±20,5 |
| | Lact N | 21,9 ±7,4 | 38,8 ±9,4 | 42,3 ±7,5 | 72,6 ±7,4 | 61,9 ±8,1 | 24,7 ±6,2 | 33,9 ±8,2 | 122,3 ±22,6 |
| | Vit D3 | 42,5 ±6,8 | 98,7 ±9,5 | 58,5 ±9,3 | 83,6 ±6,3 | 74,9 ±9,6 | 18,3 ±7,4 | 30,1 ±8,9 | 148.6 ±24,1 |
| | Lact + Vit D3 | 11,3 ±6,3 | 41,9 ±7,4 | 30,4 ±7,6 | 64,8 ±7,3 | 51,0 ±9,5 | 26,9 ±8,3 | 42,9 ±8,4 | 95,3 ±19,3 |
| **UICC II (n=6)** | ohne Zusatz | 58,1 ±8,6 | 113,2 ±5,8 | 103,9 ±11,6 | 223,4 ±8,4 | 113,5 ±12,6 | 10,4 ±3,6 | 17,1 ±9,7 | 261,7 ±27,3 |
| | Lact N | 20,8 ±7,5 | 76,3 ±6,4 | 57,3 ±10,7 | 121,7 ±10,2 | 69,4 ±10,4 | 20,9 ±4,2 | 36,6 ±9,5 | 186,7 ±32,3 |
| | Vit D3 | 34,8 ±7,9 | 102,8 ±8,9 | 77,3 ±11,1 | 169,1 ±11,1 | 80,1 ±12,8 | 18,1 ±3,1 | 27,9 ±10,3 | 213.4 ±33,6 |
| | Lact + Vit D3 | 15,6 ±6,1 | 81,6 ±5,9 | 46,2 ±8,6 | 86,3 ±9,3 | 59,6 ±9,7 | 23,9 ±3,8 | 39,3 ±7,4 | 101,8 ±47,2 |
| **UICC III (n=6)** | ohne Zusatz | 175,0 ±10,1 | 95,4 ±11,7 | 258,9 ±8,3 | 362,8 ±10,7 | 305,7 ±21,8 | 7,8 ±4,3 | 10,1 ±4,9 | 334,8 ±30,4 |
| | Lact N | 147,5 ±9,5 | 73,5 ±9,4 | 165,0 ±10,9 | 293,1 ±11,4 | 175,3 ±23,6 | 20,1 ±3,5 | 31,4 ±4.5 | 249,9 ±44,2 |
| | Vit D3 | 169,4 ±14,7 | 91,7 ±7,6 | 215,9 ±9,4 | 331,5 ±11,9 | 214,7 ±19,4 | 17,9 ±7,8 | 26,1 ±5,8 | 299,1 ±61,1 |
| | Lact + Vit D3 | 118,1 ±7,8 | 76,7 ±4,3 | 150,1 ±10,8 | 264,7 ±10,5 | 92,9 ±19,7 | 22,9 ±6,3 | 35,8 ±6,7 | 183,5 ±33,2 |
| **UICC IV (n=6)** | ohne Zusatz | 372,8 ±15,6 | 213,2 ±11,1 | 335,7 ±9,5 | 423,4 ±9,3 | 532,2 ±14,2 | 5,0 ±3,1 | 9,5 ±5,3 | 895,6 ±55,2 |
| | Lact N | 351,9 ±11,9 | 201,3 ±10,9 | 268,7 ±9,0 | 400,7 ±10,6 | 461,3 ±11,3 | 10,1 ±3,8 | 18,9 ±6,9 | 871,4 ±38,5 |
| | Vit D3 | 369,9 ±9,4 | 213,1 ±8,6 | 292,5 ±9,4 | 413,1 ±11,5 | 499,9 ±16,9 | 7,9 ±4,2 | 9,1 ±6,4 | 896,4 ±58,2 |
| | Lact + Vit D3 | 351,8 ±10,4 | 205,4 ±9,3 | 205,8 ±10,1 | 402,1 ±10,9 | 432,1 ±14,2 | 18,6 ±4,9 | 18,1 ±6,1 | 873,5 ±66,1 |

**Tabelle 2:**

| **Gesamtanteile:** | |
|---|---|
| Protein | 70-90 Gew.% oder >80 Gew.% |
| Fett | < 2 Gew.% |
| Feuchtigkeit | < 5 Gew.% |
| Laktose | < 11 Gew.% |
| | |

| **Besondere Bestandteile schon in den Gesamtanteilen vorhanden:** | |
|---|---|
| Immunoglobulin G (gemessen mit HPLC) | >20 Gew.% (bezogen auf Proteinanteil) |
| Lactoferrin | 1-5 Gew.% |
| | |

| **Weitere Bestandteile:** | |
|---|---|
| Vitamin D | <0,2 µg / 100 g |
| Eisen | <2 mg / 100 g |

**Tabelle 3:**

| **Gesamtanteile:** | |
|---|---|
| Protein | 80,7 Gew.% |
| Fett | 1,5 Gew.% |
| Feuchtigkeit | 3,9 Gew.% |
| Asche | 5,3 Gew.% |
| Laktose | 8,6 Gew.% |
| | |

| **Besondere Bestandteile schon in den Gesamtanteilen vorhanden:** | |
|---|---|
| Immunoglobulin G (gemessen mit HPLC) | 21,1 Gew.% |
| Lactoferrin | 3,1 Gew.% |
| | |

| **Weitere Bestandteile:** | |
|---|---|
| Vitamin D | <0,2 µg / 100g |
| Vitamin A | 20 µg/100g |
| B2 | 578 µg/100g |
| B6 | ND µg/100g |
| B12 | 17,8 µg/100g |
| C | 1 mg/100g |
| E | 164 µg/100g |
| Folsäure | 230 µg/100g |
| Eisen | 0,8 mg / 100 g |
| | |

| **Physikalische Eigenschaften:** | |
|---|---|
| Dichte | 0,5 g/ml |

### zitierte Literatur

### zitierte Patentliteratur

EP 0917467 A1
EP 2121002 A1

### zitierte Nicht-Patentliteratur

Cesarone, M.R., Belcaro, G., Di Renzo, A., Dugall, M., Cacchio, M., Ruffini, I., Pellegrini, L., (...), Vinciguerra, G.: "Prevention of influenza episodes with colostrum compared with vaccination in healthy and high-risk cardiovascular subjects: The epidemiologic study in San Valentino" in: Clinical and Applied Thrombosis/Hemostasis 13 (2) / 2007, pp. 130-136
Tacket CO, Binion SB, Bostwick E, Losonsky G, Roy MJ, Edelman R.: "Efficacy of bovine milk immunoglobulin concentrate in preventing illness after Shigella flexneri challenge". American Journal Tropical Medicine Hygiene 47(3) / 1992, pp.276-83
HI Huppertz et al.: "Bovine Colostrum ameliorates Diarrhea in Infection with Diarrheagenic Escherichia coli-Shiga-Toxin-Prducing E.coli, and E.coli Expressing Intimin and Hemolysin". J.Ped.Gastroenterol. Nutr. 29:452-456 1999
Uruakpa FO, Ismond MAH, Akobundu ENT: "Colostrum and its benefits: a review". Nutrition Research 22 / 2002, pp.755-67
Harmsen, Martin C. et al.: Antiviral effects of plasma and milk proteins: lactoferrin shows potent activity against both human immunodeficiency virus and human cytomegalovirus replication in vitro. Journal of Infectious Diseases, Band 172, Nr. 2, 1995, S. 380-388
Park, Ji-Hye et al.: An antimicrobial protein, lactoferrin exists in the sweat: proteomic analysis of sweat. Experimental Dermatology, Band 20, Nr. 4, 2011, S. 369-371
W.G. Struff and G. Sprotte: Bovine colostrum as a biologic in clinical medicine: a review. International Journal of Clinical Pharmacology and Therapeutics, Vol. 45 - No. 4/2007 (193-202)
W.G. Struff and G. Sprotte: Bovine colostrum as a biologic in clinical medicine: A review - Part II: International Jour nal of Clinical Pharmacology and Therapeutics, Vol. 46 - No. 5/2008 (211-225)
A.M. Waaga-Gasser, M. Gasser, M. Stock, M. Grimm and G. Sprotte: Oral immunoglobulin induces mononuclear cell apoptosis in patients suffering from idiopathic chronic pain syndrome: Results from a pilot study. International Journal of Clinical Pharmacology and Therapeutics, Vol. 47 - No. 7/2009 (421-433)
Edwin Bölke, Peter M. Jehle, Frieder Hausmann, Armin Däubler, Heidemarie Wiedeck, Gerald Steinbach, Martin Storck and Klaus Orth: PREOPERATIVE ORAL APPLICATION OF IMMUNOGLOBULIN-ENRICHED COLOSTRUM MILK AND MEDIATOR RESPONSE DURING ABDOMINAL SURGERY. SHOCK, Vol. 17, No. 1, pp. 9-12, 2002
Florian Fitzal, Ildikó Racz, János Hamar, Heinz Redl and Soheyl Bahrami: Immunoglobulin Enriched Colostral Milk Reduces Gut-Derived Endotoxemia in a Rat Hemorrhage Model. European Journal of Trauma, 2001, pp. 257-263
N. ROOS, S. MAHÉ,R. BENAMOUZIG, H. SICK, J. RAUTEREAU AND D. TOMÉ: 15N-Labeled Immunoglobulins from Bovine Colostrum Are Partially Resistant to Digestion in Human Intestine. The Journal of Nutrition, 1995, pp. 1238-1244
R. Lissner, P.A. Thürmann, G. Merz and H. Karch: Antibody reactivity and fecal recovery of bovine immunoglobulins following oral administration of a colostrum concentrate from cows (Lactobin) to healthy volunteers. International Journal of Clinical Pharmacology and Therapeutics., Vol. 36, No. 5 - 1998 (239-245)
Stubbins RE, Hakeem A, Nunez NP 2012. Using components of the Vitamin D pathway to prevent and treat colon cancer. Nutrition Reviews 70(12): 721-729
Manson J E. 2011. Vitamin D and Prevention of Cancer - Ready for Prime Time?. NEJM 364(15): 1385-87
Pietschmann P. et al. 2003. Bedeutung von Vitamin D im Immunsystem. J Mineral Stoffwechsel 10(3): 13-15
BMJ Research. Association between pre-diagnostic circulating Vitamin D concentration and risk of colorectal cancer in European Populations: A nested case-control **StudyBMI/OnlineFirst/bmj.com**
Haussler MR et al. Vitamin D receptor: molecular signaling and actions of nutritional ligands in disease prevention. Nutritional Reviews 2008; 66:98-112
A.M. Keech: Peptide Immuntherapy - Colostrum - A Physician's Reference Guide. AKS Publishing 2010
Schupp N, Schmid U, Heidland A, Stopper H. Rosuvastatin protects against oxidative stress and DNA damage in vitro via upregulation of glutathione synthesis. Atherosclerosis 2008; 199: 278-287
Guideline on Validation of the Limulus Amebocyte Lysate Test as an End-Product Endotoxin Test for Human and Animal Parenteral Drugs, Biological Products and Medical Devices. U.S. Dept. of Health & Human Services, FDA, December 1987
Interim Guidance for Human and Veterinary Drug Products and Biologicals. U. S. Department of Health & Human Services, FDA, July 15, 1991
"Bacterial Endotoxins Test." In The U.S. Pharmacopeia, 25th revision, 12601 Twinbrook Parkway, Rockville, MD 20852

## Patentansprüche

1. Pharmazeutische Zusammensetzung zur Prophylaxe und/oder zur Behandlung von Tumoren des Gastrointestinaltraktes umfassend:
a) Kolostrum und
b) Vitamin D.

2. Pharmazeutische Zusammensetzung nach Anspruch 1, **dadurch gekennzeichnet, dass** das Vitamin D Cholecalciferol (Vitamin D3) ist.

3. Pharmazeutische Zusammensetzung nach Anspruch 1 oder 2, **dadurch gekennzeichnet, dass** die pharmazeutische Zusammensetzung ca. 0,00001-0,5 Gew.%, bevorzugt 0,00002 bis 0,4 Gew.-%, besonders bevorzugt zwischen 0,00002 bis 0,3 Gew.-%. Vitamin D, vorzugsweise Cholecalciferol (Vitamin D3), enthält.

4. Pharmazeutische Zusammensetzung nach einem der vorhergehenden Ansprüche, **dadurch gekennzeichnet, dass** das Kolostrum von Säugetieren ausgewählt aus der Gruppe der Einhufer (*Equus*) oder Paarhufer (*Artiodactyla* oder *Paraxonia*) stammt.

5. Pharmazeutische Zusammensetzung nach Anspruch 4, **dadurch gekennzeichnet, dass** das Kolostrum von Wiederkäuern (Ruminantia) stammt.

6. Pharmazeutische Zusammensetzung nach Anspruch 5, **dadurch gekennzeichnet, dass** das Kolostrum von Rindern (Bovini) stammt.

7. Pharmazeutische Zusammensetzung nach einem der vorhergehenden Ansprüche, **dadurch gekennzeichnet, dass** das Kolostrum aus mindestens 100, vorzugsweise mindestens 200, Einzelkolostren stammt.

8. Pharmazeutische Zusammensetzung nach Anspruch 6 oder 7, **dadurch gekennzeichnet, dass** das Kolostrum als Konzentrat, vorzugsweise als Pulver, vorliegt.

9. Pharmazeutische Zusammensetzung nach einem der Ansprüche 6 bis 8, **dadurch gekennzeichnet, dass** das Kolostrum 70-90 Gew.% Protein und weniger als 5 Gew.% Fett enthält.

10. Pharmazeutische Zusammensetzung nach einem der Ansprüche 6 bis 9, **dadurch gekennzeichnet, dass** das Kolostrum 70-90 Gew.% Protein, weniger als 5 Gew.% Fett und weniger als 0,2 µg/100 g Vitamin D, vorzugsweise Cholecalciferol (Vitamin D3), enthält.

11. Pharmazeutische Zusammensetzung nach einem der Ansprüche 6 bis 9, **dadurch gekennzeichnet, dass** das Kolostrum 75-90 Gew.% Protein, weniger als 2 Gew.% Fett und weniger als 0,2 µg/100 g Vitamin D, vorzugsweise Cholecalciferol (Vitamin D3), enthält.

12. Pharmazeutische Zusammensetzung nach einem der Ansprüche 9 bis 11, **dadurch gekennzeichnet, dass** das Protein 20-30 Gew.% IgG, vorzugsweise IgG der Subklasse IgG1 und 1-5 Gew.% Lactoferrin bzw. Lactotransferrin enthält.

13. Pharmazeutische Zusammensetzung nach einem der vorhergehenden Ansprüche, **dadurch gekennzeichnet, dass** es sich bei den Tumoren des Gastrointestinaltraktes um kolorektale Karzinome (CRC) handelt.

14. Pharmazeutische Zusammensetzung zur Verwendung in der Prophylaxe und/oder Behandlung von Tumoren des Gastrointestinaltraktes nach einem der Ansprüche 1 bis 13.

15. Pharmazeutische Zusammensetzung zur Verwendung in der Prophylaxe und/oder Behandlung nach Anspruch 14, **dadurch gekennzeichnet, dass** es sich bei den Tumoren des Gastrointestinaltraktes um kolorektale Karzinome handelt.

## Claims

1. Pharmaceutical composition for the prophylaxis and/or for the treatment of tumours of the gastrointestinal tract, comprising:
a) colostrum and
b) vitamin D.

2. Pharmaceutical composition according to Claim 1, **characterized in that** the vitamin D is cholecalciferol (vitamin D3).

3. Pharmaceutical composition according to Claim 1 or 2, **characterized in that** the pharmaceutical composition contains approx. 0.00001 - 0.5% by weight, preferably from 0.00002 to 0.4% by weight, particularly preferably between 0.00002 to 0.3% by weight, of vitamin D, preferably cholecalciferol (vitamin D3).

4. Pharmaceutical composition according to any of the preceding claims, **characterized in that** the colostrum comes from mammals selected from the group of the solidungulates (*Equus*) or even-toed ungulates (*Artiodactyla* or *Paraxonia*).

5. Pharmaceutical composition according to Claim 4, **characterized in that** the colostrum comes from ruminants (Ruminantia).

6. Pharmaceutical composition according to Claim 5, **characterized in that** the colostrum comes from cattle (Bovini).

7. Pharmaceutical composition according to any of the preceding claims, **characterized in that** the colostrum comes from at least 100, preferably at least 200, individual colostra.

8. Pharmaceutical composition according to Claim 6 or 7, **characterized in that** the colostrum is present as a concentrate, preferably as a powder.

9. Pharmaceutical composition according to any of Claims 6 to 8, **characterized in that** the colostrum contains 70-90% by weight of protein and less than 5% by weight of fat.

10. Pharmaceutical composition according to any of Claims 6 to 9, **characterized in that** the colostrum contains 70-90% by weight of protein, less than 5% by weight of fat and less than 0.2 µg/100 g vitamin D, preferably cholecalciferol (vitamin D3) .

11. Pharmaceutical composition according to any of Claims 6 to 9, **characterized in that** the colostrum contains 75-90% by weight of protein, less than 2% by weight of fat and less than 0.2 ug/100 g vitamin D, preferably cholecalciferol (vitamin D3) .

12. Pharmaceutical composition according to any of Claims 9 to 11, **characterized in that** the protein contains 20-30% by weight of IgG, preferably IgG of the IgG1 subclass, and 1-5% by weight of lactoferrin or lactotransferrin.

13. Pharmaceutical composition according to any of the preceding claims, **characterized in that** the tumours of the gastrointestinal tract are colorectal carcinomas (CRCs).

14. Pharmaceutical composition for use in the prophylaxis and/or treatment of tumours of the gastrointestinal tract according to any of Claims 1 to 13.

15. Pharmaceutical composition for use in the prophylaxis and/or treatment according to Claim 14, **characterized in that** the tumours of the gastrointestinal tract are colorectal carcinomas.

## Revendications

1. Composition pharmaceutique pour la prophylaxie et/ou le traitement de tumeurs du tractus gastro-intestinal, comprenant :
a) du colostrum et
b) de la vitamine D.

2. Composition pharmaceutique selon la revendication 1, **caractérisée en ce que** la vitamine D est le cholécalciférol (vitamine D3).

3. Composition pharmaceutique selon la revendication 1 ou 2, **caractérisée en ce que** la composition pharmaceutique contient environ 0,00001 à 0,5 % en poids, de préférence 0,00002 à 0,4 % en poids, de manière particulièrement préférée 0,00002 à 0,3 % en poids de vitamine D, de préférence de cholécalciférol (vitamine D3).

4. Composition pharmaceutique selon l'une quelconque des revendications précédentes, **caractérisée en ce que** le colostrum est issu de mammifères choisis dans le groupe des équidés (*Equus*) ou des artiodactyles (*Artiodactyla* ou *Paraxonia*).

5. Composition pharmaceutique selon la revendication 4, **caractérisée en ce que** le colostrum est issu de ruminants (Ruminantia).

6. Composition pharmaceutique selon la revendication 5, **caractérisée en ce que** le colostrum est issu de bovins (Bovini).

7. Composition pharmaceutique selon l'une quelconque des revendications précédentes, **caractérisée en ce que** le colostrum est issu d'au moins 100, de préférence d'au moins 200 colostrums individuels.

8. Composition pharmaceutique selon la revendication 6 ou 7, **caractérisée en ce que** le colostrum se présente sous la forme d'un concentré, de préférence sous la forme d'une poudre.

9. Composition pharmaceutique selon l'une quelconque des revendications 6 à 8, **caractérisée en ce que** le colostrum contient 70 à 90 % en poids de protéines et moins de 5 % en poids de matières grasses.

10. Composition pharmaceutique selon l'une quelconque des revendications 6 à 9, **caractérisée en ce que** le colostrum contient 70 à 90 % en poids de protéines, moins de 5 % en poids de matières grasses et moins de 0,2 µg/100 g de vitamine D, de préférence de cholécalciférol (vitamine D3).

11. Composition pharmaceutique selon l'une quelconque des revendications 6 à 19, **caractérisée en ce que** le colostrum contient 75 à 90 % en poids de protéines, moins de 2 % en poids de matières grasses et moins de 0,2 µg/100 g de vitamine D, de préférence de cholécalciférol (vitamine D3).

12. Composition pharmaceutique selon l'une quelconque des revendications 9 à 11, **caractérisée en ce que** les protéines contiennent 20 à 30 % en poids d'IgG, de préférence d'IgG de la sous-classe IgG1, et 1 à 5 % en poids de lactoferrine ou de lactotransferrine.

13. Composition pharmaceutique selon l'une quelconque des revendications précédentes, **caractérisée en ce que** les tumeurs du tractus gastro-intestinal sont des cancers colorectaux (CCR).

14. Composition pharmaceutique destinée à une utilisation pour la prophylaxie et/ou le traitement de tumeurs du tractus gastro-intestinal selon l'une quelconque des revendications 1 à 13.

15. Composition pharmaceutique destinée à une utilisation pour la prophylaxie et/ou le traitement selon la revendication 14, **caractérisée en ce que** les tumeurs du tractus gastro-intestinal sont des cancers colorectaux.
